# EUROPEAN PATENT APPLICATION

(11) **EP 4 458 378 A1**
(43) Date of publication of application: **06.11.2024**
(21) Application number: 22915109.7
(22) Date of filing: 29.12.2022
(51) Int. Cl.: A61K 48/00, A61K 35/17, A61K 39/39, C07K 14/73, C12N 15/11, A61P 35/00

(54) **PREPARATION TECHNIQUE FOR UNIVERSAL CAR-T CELL, AND APPLICATION OF UNIVERSAL CAR-T CELL**

(30) Priority: 30.12.2021 CN 202111652734; 30.12.2021 CN 202111652757; 30.12.2021 CN 202111657691; 30.12.2021 CN 202111657717
(71) Applicant: Chongqing Precision Biotech Co., Ltd., Chongqing 400039 (CN); Chongqing Precision Biological Industrial Technology Research Institute Co., Ltd., Chongqing 400039 (CN)
(72) Inventor: LI, Wuling, Chongqing 400039 (CN); XU, Yanmin, Chongqing 400039 (CN); SHEN, Junjie, Chongqing 400039 (CN); YANG, Zhi, Chongqing 400039 (CN)
(74) Representative: dompatent von Kreisler Selting Werner - Partnerschaft von Patent- und Rechtsanwälten mbB
(86) International application number: PCT/CN2022/143615
(87) International publication number: WO 2023/125860

(57) **Abstract**

A preparation method for a universal CAR-T cell, and an use of a universal CAR-T cell. When universal CAR-T is prepared, an RNP complex comprises an RNP complex composed of a Cas9 protein and sgRNA; and the SgRNA is SgRNA of CD4 and/or sgRNA of CD8. The CD4 gene and/or CD8 gene of the universal CAR-T cell or a T cell are/is knocked out and/or inactivated. The CAR-T cell expresses CD44 and/or CD62L, so that the activity of the CAR-T cell can be enhanced, the effectiveness of CAR-T is enhanced, the CAR-T cell has a better phenotype and lower exhaustion expression, and the killing of the CAR-T cell against a tumor target cell is improved, and thus, the CAR-T cell can be used for targeted therapy of tumors and can be used for allogenic CAR-T therapy.

## Description

The present application claims the benefit of priority to the Chinese Patent Application No. 202111652734.4 field to China National Intellectual Property Administration on December 30, 2021 and entitled *Preparation Technology and Use of Universal CAR-T Cell*, which is incorporated herein by reference in its entirety.

The present application claims the benefit of priority to the Chinese Patent Application No. 202111652757.5 field to China National Intellectual Property Administration on December 30, 2021 and entitled *Preparation Technology and Use of Universal CAR-T Cell*, which is incorporated herein by reference in its entirety.

The present application claims the benefit of priority to the Chinese Patent Application No. 202111657691.9 field to China National Intellectual Property Administration on December 30, 2021 and entitled *Preparation Method and Use of Universal CAR-T Cell*, which is incorporated herein by reference in its entirety.

The present application claims the benefit of priority to the Chinese Patent Application No. 202111657717.X field to China National Intellectual Property Administration on December 30, 2021 and entitled *Preparation Method and Use of Universal CAR-T Cell*, which is incorporated herein by reference in its entirety.

### TECHNICAL FIELD

The present disclosure belongs to the technical fields of cell biology, molecular biology and drug research and development, and particularly relates to a preparation method for a universal CAR-T cell, and an use of a universal CAR-T cell.

### BACKGROUND

CAR-T immunotherapy involves modifying a patient's autologous T lymphocytes through gene engineering to enable the T lymphocytes to express chimeric antigen receptors, which can effectively overcome immune escape of tumor cells by downregulating expression of MHC molecules and reducing antigen presentation, leaving the tumor cells with no place to escape. Currently, the two T cell products with chimeric antigen receptors (CARs) approved for in China are derived from autologous T cells, but these autologous CAR-T cells approved for clinical use must be made on a customized basis. However, expensive production costs and lengthy manufacturing process remain significant factors for autologous T cells production platforms to promote large-scale clinical use, moreover, there are risks such as production failure . In addition, the production process of personalized and customized autologous CAR-T cells also restricts extensive use of the autologous T cells in different tumor types. Therefore, universal allogenic T cells are needed to prepare universal CAR-T cells, and these cells serve as "off-the-shelf' ready-to-use therapeutic agents for large-scale clinical use. The universal CAR-T cells can significantly shorten treatment cycles and reduce costs. Problems related to graft rejection reactions and efficacy can be gradually solved through strategies such as gene editing technology and donor-derived T cells for stem cell transplant. Therefore, they are more competitive in the future market.

The universal CAR-T cells are a kind of living cell agents for allogeneic transplantation, for which T cells derived from peripheral blood of a healthy donorare taken as starting samples, gene editing technology is adopted to solve the problem of immunological rejection among allogeneic, and the T cells are loaded with CAR structures specifically targeting cancer targets, and are finally used to transfuse to an allogeneic cancer patient for specifically targeting tumor cells.

Genome editing technologies, including zinc finger nuclease (ZFN), transcription activator-like effect nuclease (TALEN) and CRISPR-related technologies, are adopted to prepare universal T cells. At present, conventional operation methods in the industry are much the same, that is, TCR molecules and/or MHC-related molecules in the allogenic T cells that may cause strong immune rejection reactions are knocked out, to solve the problem of immune rejection. In addition, in order to improve the therapeutic effect or safety, most institutions further knock out PD-1, CTLA-4, CD52 or introduce CAR suicide gene switches on the basis of gene editing.

The operation methods are theoretically feasible, but no literature that analyze in detail the impact of the T cells without the TCR or MHC molecules on cell survival and function is available at present, which is also one of the reasons that the industry currently faces a great dilemma.

At present, the industry faces a considerable difficulty that the universal CAR-T cells have poor in vivo survival, and increasing dose of transfusion or number of transfusion are even incapable of achieving a desired therapeutic effect. However, relatively low does of the autologous CAR-T cells came with higher response rate, which may result from many factors. TCR serves as a key gene for T cells to perform various immune-related functions, but no studies clearly prove whether the TCR plays a critical or decisive auxiliary role in other functions of T cells. However, it is impossible for the autologous CAR-T cells to have advantages of the universal CAR-T cells, such as being ready-to-use, off-the-shelf, and low treatment costs. Therefore, it is necessary to prepare a universal CAR-T with long duration of in vivo survival.

### SUMMARY

In view of this, the present disclosure provides a preparation technique for a universal CAR-T cell, and an use of a universal CAR-T cell.

In order to achieve the above objectives, the present disclosure provides the following technical solutions:

A first objective of the present disclosure is to provide an RNP complex, and the complex can be used to knock out target sites in the T cell.

In order to achieve the above objectives, the technical solution of the present invention is:

an RNP complex, the RNP complex is an RNP complex composed of a Cas9 protein and sgRNA; and the sgRNA is sgRNA of CD4 and/or sgRNA of CD8.

Further, the RNP complex is an intracellular working form of CRISPR/Cas9, and an extracellular delivery mode includes the following combinations: a plasmid delivery mode, a virus delivery mode, an mRNA (Cas9) and gRNA delivery mode, an mRNA (Cas9) and plasmid (gRNA) delivery mode, a virus (Cas9) and gRNA delivery mode, a virus (Cas9) and plasmid (gRNA) delivery mode, a plasmid (Cas9) and virus (gRNA) delivery mode, a plasmid (Cas9) and a gRNA delivery mode.

Further, in the RNP complex, CD4 gene is CD4 gene on human chromosome 12: 6,789,528-6,820,799 forward strand; and CD8 gene is CD8A gene on human chromosome 2: 86,784,610-86,808,396 reverse strand, and is CD8B gene on human chromosome 2: 86,815,339-86,861,924 reverse strand.

Further, a sgRNA sequence of the CD4 is shown as any one of SEQ ID NO. 1 -6 or SEQ ID NO. 14 -24; and a sgRNA sequence of the CD8 is shown as any one of SEQ ID NO. 7 -11 or SEQ ID NO. 25 -33.

A second objective of the present disclosure is to provide a T cell with a long in-vivo survival, and the T cell can be used to further prepare a CAR-T cell.

In order to achieve the above objectives, the present disclosure adopts the following technical solution:

a T cell, CD4 gene and/or CD8 gene of the T cell are/is knocked out and/or inactivated.

Some of the target sequences used in the present disclosure are shown as SEQ ID NO. 1-11, the sequences are not limited to the sequences shown herein, and include CD4 gene on human chromosome 12: 6,789,528-6,820,799 forward strand, CD8A gene on human chromosome 2: 86,784,610-86,808,396 reverse strand, and CD8B gene on human chromosome 2: 86,815,339-86,861,924 reverse strand; and Table 1 of target locations for gene editing show target sequences in any region that could cause deletion of gene itself.

Further, the T cell is obtained through gene editing.

Further, the T cell is obtained through natural separation.

Further, the T cell disables or deletes of β2-microglobulin, and/or a common subunit of an HLA-1 molecule, and/or CIITA gene.

Further, the T cell is an allogeneic T cell.

Further, the T cell also expresses HLA-E and/or HLA-G and/or HLA-F proteins.

Further, the T cell, the T cell expresses CD44.

Further, as an optimization, the T cell also expresses CD62L gene.

Further, amino acid sequences of the CD62L gene in the T cell are shown as SEQ ID NO. 13-17.

Further, in the T cell, an amino acid sequence of the CD44 is shown as SEQ ID NO. 12, and an amino acid sequence of the CD62L is shown as any one of SEQ ID NO. 13.

Further, in the T cell, the CD4 gene and/or CD8 gene of the T cell are/is knocked out and/or inactivated. An amino acid sequence of the CD44 gene is shown as SEQ ID NO. 12; partial target sequences of the CD4 gene and the CD8 are shown as SEQ ID NO. 1 -11, the sequences are not limited to the sequences shown herein, and include CD4 gene on human chromosome 12: 6,789,528-6,820,799 forward strand, CD8A gene on human chromosome 2: 86,784,610-86,808,396 reverse strand, and CD8B gene on human chromosome 2: 86,815,339-86,861,924 reverse strand; and Table 1 and Tables 3-5 of target locations for gene editing show target sequences in any region that could cause deletion of gene itself. Further, in order to improve knockout efficiency, one guanine (G) can be added before a first base of a target sequence that is non-G sequence.

A natural amino acid sequence of CD62L is shown as SEQ ID NO. 13.

Since natural or natural sequence-expressed CD62L has a mode of self-cleavage that results in degradation or hydrolysis under specific physiological conditions (when an activation degree is relatively high), it can be mutated into the following non-degradable mode sequence in a manner such as amino acid substitution, deletion or other truncation, so as to enhance its persistence.

**Table 1**

| Sequence ID | Name of | Sequence |
|---|---|---|
| | Sequence | |
| SEO ID NO.1 | sg4-1 | TGAACCGGGGAGTCCCTTTT |
| SEO ID NO.2 | sg4-2 | CCCTTTTAGGCACTTGCTTC |
| SEO ID NO.3 | sg4-3 | AGTGGTGCTGGGCAAAAAAG |
| SEO ID NO.4 | sg4-4 | TCTGGTTGGAGTTTTTCCAG |
| SEO ID NO.5 | sg4-5 | TCAGGGAAAGAAAGTGGTGC |
| SEO ID NO.6 | sg4-6 | CTCCTCCCAGCAGCCACTCA |
| SEO ID NO.7 | sg8-1 | GACCGCCTTGCTCCTGCCGC |
| SEO ID NO.8 | sg8-2 | GGCAGGAGCAAGGCGGTCAC |
| SEO ID NO.9 | sg8-3 | TCACGGAGCAGCAAGGCCAG |
| SEO ID NO.10 | sg8-4 | GGCCAGCGGCAGGAGCAAGG |
| SEO ID NO.11 | sg8-5 | CAAGGCCAGCGGCAGGAGCA |
| SEO ID NO.12 | CD44 | MDKFWWHAAWGLCLVPLSLAQIDLNITCRFAGVFHVE KNGRYSISRTEAADLCKAFNSTLPTMAQMEKALSIGFE TCRYGFIEGHVVIPRIHPNSICAANNTGVYILTSNTSQYD TYCFNASAPPEEDCTSVTDLPNAFDGPITITIVNRDGTR YVQKGEYRTNPEDIYPSNPTDDDVSSGSSSERSSTSGG YIFYTFSTVHPIPDEDSPWITDSTDRIPATTLMSTSATATE TATKRQETWDWFSWLFLPSESKNHLHTTTQMAGTSSN TISAGWEPNEENEDERDRHLSFSGSGIDDDEDFISSTIST TPRAFDHTKQNQDWTQWNPSHSNPEVLLQTTTRMTD VDRNGTTAYEGNWNPEAHPPLIHHEHHEEEETPHSTSTI QATPSSTTEETATQKEQWFGNRWHEGYRQTPKEDSHST TGTAAASAHTSHPMQGRTTPSPEDSSWTDFFNPISHPM GRGHQAGRRMDMDSSHSITLQPTANPNTGLVEDLDRT GPLSMTTQQSNSQSFSTSHEGLEEDKDHPTTSTLTSSNR NDVTGGRRDPNHSEGSTTLLEGYTSHYPHTKESRTFIP VTSAKTGSFGVTAVTVGDSNSNVNRSLSGDQDTFHPSG GSHTTHGSESDGHSHGSQEGGANTTSGPIRTPQIPEWLII LASLLALALILAVCIAVNSRRRCGQKKKLVINSGNGAVE DRKPSGLNGEASKSQEMVHLVNKESSETPDQFMTADE TRNLQNVDMKIGV |
| SEO ID NO.13 | CD62L Native sequence | |

**Table 2**

| Gene | Exon No. | Sequence position |
|---|---|---|
| CD4 | ENSE00003580321 | 63-116 bp |
| | ENSE00003508863 | 1-165 bp |
| | ENSE00003497892 | 1-159 bp |
| CD8A | ENSE0000 1897263 | 89-137 bp |
| | ENSE00001220182 | 1-354 bp |
| | ENSE00001011446 | 1-111 bp |
| CD8B | ENSE00001761743 | 79-93 bp |
| | ENSE00002506201 | 1-360 bp |
| | ENSE00002475314 | 1-90 bp |

A third objective of the present disclosure is to provide a method for preparing the above T cell, and the method provides a preparation idea for providing a cell with long persistence.

In order to achieve the above objectives, a technical solution of the present disclosure is:
the RNP complex is adopted to knock out and/or inactivate the CD4 gene and/or CD8 gene in a T lymphocyte through gene editing technology.

For the method for preparing the T cell, any one of the following methods is used to obtain a target cell:
(1) a T cell from a fresh or cryopreservation source, CD4 and CD8 genes are knocked out or inactivated through gene editing, the CD4 and CD8 genes can be simultaneously knocked out or inactivated, alternatively, the CD4 or CD8 gene can be first knocked out or inactivated, and the CD8 or CD4 can be further knocked out or inactivated; and the target cell is finally obtained; (2) a CD4-positive T cell is sorted out, and CD4 gene is knocked out or inactivated through gene editing to obtain the target cell; (3) a CD8-positive T cell is sorted out, and CD8 gene is knocked out or inactivated through gene editing to obtain the target cell; (4) a CD4-positive T cell is sorted out, and CD4 gene is knocked out or inactivated through gene editing; a CD8-positive T cell is sorted out, and CD8 gene is knocked out or inactivated through gene editing; and the T cell of which the CD4 gene has been knocked out or inactivated through gene editing and the T cell of which the CD8 gene knocked out or inactivated through gene editing are mixed to obtain the target cell.

Further, the T cell is an activated and/or non-activated T lymphocyte.

Further, timing for knocking out or inactivating the CD4 gene and/or CD8 gene in the T cell is: after amplification of the T cell, or before amplification of the T cell.

Further, the gene editing technology is to modify the T cell through electroporation or non-electroporation.

Further, the gene editing technology includes CRISPR/(CAS 9 or Cpf1), ZFN, TALEN.

A fourth objective of the present disclosure is to provide a universal CAR-T cell in vivo featuring longer persistence, where the universal CAR-T cell can express CD44 and/or CD62L.

Protein encoded by the CD44 gene is a cell surface glycoprotein, which participates in intercellular interaction, cell adhesion and migration. It is a receptor of hyaluronic acid (HA), and can also interact with other ligands, such as osteopontion, collagen, and matrix metalloproteinase (MMP). Various cellular functions participated in by the proteins include lymphocyte activation, recycling and homing, hematopoiesis and tumor metastasis. Current studies on CD44 mainly focus on its role in tumor stem cells and effects of different variants thereof in cell adhesion, and very few studies use CD44 as a target for CAR-T cell therapy, and there has been no relevant report that directly apply the CD44 to the CAR-T therapy.

L-selectin (CD62L) gene encodes a cell surface adhesion molecule belonging to an adhesion/homing receptor family, and it is usually taken as a marker for T cell activation. In a continuous activation process of the T cell, expression thereof on cells is continuously downregulated due to initiation of its self-cleavage mechanism. Studies show that anti-tumor ability of cytotoxic T lymphocytes (CTL) expressing CD62L is significantly better than that of CTL (doi: 10.1172/JCI24480 doi: 10.1073/pnas.0503726102) without expressing CD62L even with a down-regulation phenomenon, which may benefit from the homing ability of CD62L. However, studies also indicate that the L-selectin has nothing to do with homing, but is associated with the activation of therapeutic T cells inside the tumor (doi: 10.3389/fimmu.2019.01321). An article (doi: 10.3389/Ficu. 2019.01321) uses adoptive immunotherapy in mice. The study used a mouse model of adoptive immunotherapy, which takes a wild-type mouse CD8+T as a control, and generates mutant CD62L transgenic mice by deleting partial sequences of hydrolytic cleavage sites at the proximal membrane end of CD62L, where the mutant transgenic mice enhances the expression of L-selectin on mice CD8+T cells by mutating hydrolyzed fragments thereof. Study results show that mutant CD62L transgenic mice can improve the ability of CD8+T cells to control the growth of solid tumors; however, in the prior art, the effects achieved by overexpressing CD62L on T cells or CAR-T cells, as well as the specific mechanism, remain unclear.

A fifth objective of the present disclosure is to provide a CAR-T cell with high long-term survival ability and a preparation method thereof. The above cells can be commercially applied, and the method has good reproducibility of operation.

In order to achieve the above objectives, a technical solution of the present disclosure is:
a CAR-T cell prepared from the T cell.

Further, CAR in the CAR-T cell contains an extracellular domain, a transmembrane domain, and an intracellular signal domain.

Further, the CAR-T cell is an allogeneic CAR-T cell.

Further, the CAR-T cell also expresses membrane-bound IL15.

Further, the CAR-T cell also expresses secretory cytokines, and the secretory cytokines include IL2, IL7, IL12, IL21, and IL15.

Further, the CAR-T cell also express and can recognize a fusion protein of molecules in a tumor microenvironment, and an extracellular structure of the fusion protein can recognize PD1, PDL1, SIRPy, SIRPδ and TIGIT.

Further, the CAR structure in the CAR-T cell can be regulated for activation or inactivation, and a structure for regulating activation or inactivation include Peptide neo-epitope (PNE), fluorescein (FITC), 10 amino acids (5B9 tag), FITC-HM-3 bifunctional molecule (FHBM), and ScFv, Leucine ZipFv linked to antibody, and Streptavidin 2 (mSA2) biotin-binding domain, and these molecules can be recognized by antibodies or Zipcar structure linked to the CAR, leading to induction and activation of signaling pathways of the CAR-T cell. These systems control the vitality of the CAR-T cell by changing a number of switch molecules linked to the antibodies.

Further, the CAR-T cell can recognize solid tumors, and hematological tumor cells/tissues; and the antigen recognition region of the CAR-T cell can recognize antigens, including: CD19, CD20, CD22, CD33, CLL-1 (CLEC12A), CD7, CD5, CD70, CD123, CEACAM 5, CEACAM 6, CEACAM 7, Mesothelin, MUC1,CLDN18.2, CDH17, Trop2, BCMA, NKG2D, PDL1, EGFR, EGFRVIII, PSCA, PSMA, MUC16, CD133, GD2, 1L13R2, B7H3, Her2, CD30, SLAMF7, CD38, GPC3, WT1 or TAG-72.

A sixth objective of the present disclosure is to provide a preparation method of the CAR-T cell, and any one of the following methods can be selected to obtain a target cell:
(1) collecting and obtaining the T cell, infecting the T cell with viruses containing CD44 and CAR structures, or infecting the T cell with viruses containing CD62 and CAR structures; and performing RNP electroporation of the T cell infected with the CAR structure to obtain the target cell; or
(2) collecting and obtaining the T cell, performing electroporation RNP of the T cell and then infecting the T cell with viruses containing CD44 and CAR structures, or infecting the T cell with viruses containing CD62 and CAR structures to obtain the target cell.

Further, according to the preparation method, any one of the following methods can be selected to obtain a target cell: (1) activating the T cell with CD4 and/or CD8 gene knocked out or inactivated, infecting the T cell with viruses containing CD62L, CD44 and CAR structures, and performing RNP electroporation of the T cell infected with the CAR structure to obtain the target cell; or

Further, according to the preparation method, any one of the following methods can be selected to obtain a target cell: (2) activating the T cell with CD4 and/or CD8 gene knocked out or inactivated, performing RNP electroporation of the T cell, and infecting the T cell with viruses containing CD62L, CD44 and CAR structures to obtain the target cell.

Further, in the preparation method, the T cell are modified by using CRISPR/Cas9 gene editing technology when activating the T cell with CD4 and/or CD8 gene knocked out or inactivated; and the RNP complex involved in the CRISPR/Cas9 gene editing technology is specifically an RNP complex containing CD4 sgRNA and CD8 sgRNA and Cas9 protein.

Further, in the preparation method, a molar ratio of the Cas9 protein to the sgRNA is 1: 2.

A preparation method of the CAR-T cell includes the following steps: 1) constructing a viral vector containing a target gene fragment (containing a CAR structure sequence and a CD62L gene fragment for specific tumor targets initiated by different promoters); and 2) infecting the viral vector with the T cell to obtain a target cell.

Further, in the preparation method above, expression of the CD62L gene in the step 2) is enhanced in a manner such as lentiviral infection, gene targeting, liposomal transfection, and electroporation.

A seventh objective of the present disclosure is to provide a cell composition, the cell composition aggregates a large number of functional cells, and the cell composition has a therapeutic effect.

In order to achieve the above objectives, a technical solution of the present disclosure is:
a cell composition containing the T cell and/or the CAR-T cell.

Further, a proportion of the T cell or the CAR-T cell contained in the cell composition is not less than 90%, 80%, 70% or 60% by quantity.

Further, the proportion of the T cell or the CAR-T cell contained in the cell composition is not less than 60% by quantity.

Further, the proportion of the T cell or the CAR-T cell contained in the cell composition is not less than 70% by quantity.

Further, the proportion of the T cell or the CAR-T cell contained in the cell composition is not less than 80% by quantity.

Further, the proportion of the T cell or the CAR-T cell contained in the cell composition is not less than 90% by quantity.

Further, the cell composition may be a CIK cell or a NKT cell induced by activation.

Further, T lymphocytes with the inactivated CD4 gene and CD8 gene in the cell composition express a chimeric antigen receptor.

An eighth objective of the present disclosure is to provide an use of the T cell, the recombinant vector or the CAR-T cell or the cell composition, and the use is a clinical provided treatment idea.

In order to achieve the above objectives, a technical solution of the present disclosure is:
an use of the T cell and/or the CAR-T cell and/or the recombinant vector and/or the cell composition in preparing cell therapy drugs.

Further, in the use, the T cell and/or the CAR-T cell and/or the recombinant vector and/or the cell composition are used in preparation of drugs for the treatment of acute lymphoblastic leukemia, chronic lymphocytic leukemia, chronic myelogenous leukemia, non-Hodgkin lymphoma, Hodgkin lymphoma, prostate cancer, colorectal cancer, breast cancer, ovarian cancer, cervical cancer, pancreatic cancer, lung cancer, kidney cancer, liver cancer, brain cancer and/or skin cancer.

The RNP in the present disclosure is used in drugs for cell therapy for tumor treatment.

When the therapeutic T cell or CAR-T cell is prepared, an inhibitor and a knockout agent of CD4 gene, and/or an inhibitor and a knockout agent of CD4 gene are used as a cell proliferation agent, a vitality enhancer, a phenotypic distribution improver, and a exhaustion marker expression inhibitor.

An use of CD62L and/or CD44 in preparing the vitality enhancer for the CAR-T cell.

Further, CD44 combined with CD62L is used to prepare the vitality enhancer for the CAR-T cell.

CD62L and/or CD44 are used to prepare the inhibitor of PD-1 and LAG-3 factors secreted by the CAR-T cell.

### Glossary:

When preparing the CAR-T cell, CD62L and/or CD44 genes are used as accelerators for tumor cell killing, and the CAR-T cell is the conventional CAR-T cell, the CAR-T cell or the universal CAR-T cell without expressing CD4 and/or CD8 genes.

In the present disclosure, the CAR structure can be a conventional first-generation, second-generation or third-generation CAR structure, or can be a novel CAR structure such as an improved dual CAR, a regulable CAR structure (such as FRB/FKBP12 regulation).

In the present disclosure, the CAR structure contains one or more components of a natural killer cell receptor (NKR), such that an NKR-CAR is formed. The NKR component can be a transmembrane domain, a hinge domain, or a cytoplasmic domain from any of the following natural killer cell receptors: killer cell immunoglobulin-like receptors (KIR), such as KIR2DL1, KIR2DL2/L3, KIR2DL4, KIR2DL5A, KIR2DL5B, KIR2DS1, KIR2DS2, KIR2DS3, KIR2DS4, DIR2DS5, KIR3DL1/S1, KIR3DL2, KIR3DL3, KIR2DP1 and KIR3DP1; natural cytotoxicity receptors (NCR), such as NKp30, NKp44 and NKp46; signaling lymphocytic activation molecule (SLAM) family of immune cell receptors, such as CD48, CD229, 2B4, CD84, NTB-A, CRA, BLAME and CD2F-10; Fc receptor (FcR), such as CD16 and CD64; and Ly49 receptor, such as LY49A and LY49C. The NKR-CAR molecule can interact with an adapter molecule or an intracellular signal domain (such as DAP12).

In the present disclosure, the lentiviral vector is selected from a group essentially consisting of the following viruses: human immunodeficiency virus 1 (HIV-1), human immunodeficiency virus 2 (HIV-2), visna-maedivide virus (VMV), caprine arthritis-encephalitis virus (CAEV), equine infectious anemia virus (EIAV), feline immunodeficiency virus (FIV), bovine immunodeficiency virus (BIV), and simian immunodeficiency virus (SIV).

In the present disclosure, the CAR-T can adopt the CAR-T structure disclosed in the patents 201710305078.8, 201710613317.6, 201710618293.3, 201510304389.3, 201510648252.X, 201810207761.2, 201810934637.6, 201811125906.0, 201910407001.0, 202010257762.5, 202010259458.4, 202010260805.5, 202010260849.8, 202010559366.8, 202110556147.9 and other disclosed CAR-T structures. The double-negative universal CAR-T cell therapy can be applied to any CAR structure or target, that is, it is not limited by CAR targets and structures, and the T cell expressing neither CD4 nor CD8 genes will not affect the expression of any CAR structure.

The gene editing technology used in the present disclosure includes, but is not limited to, CRISPR series technologies (CAS9, Cpf1), ZFN and TALEN, and the double-negative universal T cell described herein can be obtained through any of the gene editing technologies.

In the present disclosure, the allogeneic T cell refers to the T cell whose CD4 and CD8 genes have been inactivated by the gene editing technology, and can be used for allogeneic treatment of specific indications.

In the present disclosure, the pharmaceutical composition can be used in combination with one or more of chemotherapy agents, other tumor target monoclonal antibody drugs, antibody-drug conjugates (ADC), immune checkpoint inhibitor drugs, and small molecule drugs.

In the present disclosure, the hinge region sequence in the CAR-T structure can be derived from IgG, CD8, CD7 and CD4.

In the present disclosure, the transmembrane region sequence in the CAR-T structure can be derived from CD8, CD28, CD3ε, CD4, CD16, CD137, CD80 and CD86.

In the present disclosure, the intracellular signal region in the CAR-T structure can be derived from CD3, CD137, CD28, CD27, OX40, ICOS, GITR, CD2, CD40, PD-1, PD1L, B7-H3, lymphocyte function-associated antigen-1 (LFA-1), ICAM-1, CD7, NKG2C, CD83, CD86, and CD127.

In the present disclosure, the antigen recognition region can be a ligand/receptor for recognizing a target antigen; the ScFv can be an ScFv of a mouse antibody or an all-human antibody or a human-mouse chimeric antibody, or can be a single-domain antibody, such as a shark, llama, or camel antibody, or can be a bispecific antibody, or can be an artificially designed target-specific fibronectin type III (FN3) domain combination capable of identifying specific targets.

In the present disclosure, the CAR-T can adopt the CAR-T structure disclosed in the patents 201710305078.8, 201710613317.6, 201710618293.3, 201510304389.3, 201510648252.X, 201810207761.2, 201810934637.6, 201811125906.0, 201910407001.0, 202010257762.5, 202010259458.4, 202010260805.5, 202010260849.8, 202010559366.8, 202110556147.9 and other disclosed CAR-T structures, and the double-negative universal CAR-T cell therapy can be applied to any one of the CAR uses except the CD44 target, and is not limited by CAR targets and structures.

The activated T cell in the preparation method disclosed in the present disclosure can be infected with the CAR structure through a virus, or a transposon system can also be used.

The DU-CAR-T in the present disclosure refers to the universal CAR-T cell from which the CD4 and/or CD8 genes are knocked out.

The 44 -19 CAR in the present disclosure refers to the CAR-T cell that expresses CD44 and is prepared from the autologous immune cell.

The L-19CAR in the present disclosure refers to the CAR-T cell that expresses CD62 and is prepared from the autologous immune cell.

The activated T cell in the preparation method disclosed in the present disclosure can be infected with the CAR structure through a virus, or a transposon system can also be used.

In the present disclosure, the conventional universal CAR-T expressing CD44 prepared by using the allogeneic cell is CU-44-CAR-T; and the universal CAR-T expressing CD44 prepared by using the allogeneic cell is DU-44-CAR-T.

In the present disclosure, the conventional universal CAR-T expressing CD62L prepared by using the allogeneic cell is CU-62-CAR-T; and the universal CAR-T expressing CD62 prepared by using the allogeneic cell is DU-62-CAR-T.

The present disclosure has the beneficial effects:
1) the double-negative universal CAR-T cell provided by the present disclosure can not only be effectively expressed in the T lymphocyte, but also enhance the vitality of the CAR-T cell, the CAR-T cell has a better phenotype and lower exhaustion expression, and the killing of the CAR-T cell against a tumor target cell is improved, and the CAR-T cell can be used for targeted therapy of tumors.
2) The present disclosure provides an optional candidate cell of the universal CAR-T cell, which can effectively kill tumor target cells in vivo and in vitro, and can be used for targeted therapy of tumors, but also has anti-tumor effects better than those of the conventional autologous CAR-T cell and the conventional universal CAR-T cell.
3) The universal CAR-T cell provided in the present disclosure, like the conventional universal CAR-T cell, makes only lower GVH reaction in vitro, and can be used for the allogeneic CAR-T therapy, thereby having important guiding significance for the clinical use of CAR-T and the development of new strategies for combination therapy of tumors.
4) The DU-CAR-T prepared by the method according to the present disclosure can realize achieve the same lower level of GVH reaction of conventional universal CAR-T cells, and the 44-CAR, L-CAR and universal DU-CAR in the present disclosure are superior to or not inferior to the autologous CAR cell.
5) Compared with the natural DN-T or the DN-CAR-T cell prepared therefrom, the DU-CAR-T cell prepared by the method according to the present disclosure has stronger DU-CAR-T proliferation ability, higher vitality, better phenotypic distribution, lower expression of exhaustion markers and better in-vitro efficacy, with extremely significant statistical differences. The cell prepared according to the present disclosure is easy to obtain. With the same starting count of PBMC, the effective cell count finally obtained is far higher than the DN-CAR-T cell, which greatly reduces the production cost and provides more possibilities for clinical uses thereof.

### BRIEF DESCRIPTION OF THE DRAWINGS

FIG. 1 shows knockout rate detection results.
FIG. 2 shows CAR positive rate detection results.
FIG. 3 shows in vitro pharmacodynamic detection results.
FIG. 4 shows phenotype detection results.
FIG. 5 shows exhaustion marker detection results.
FIG. 6 shows vitality monitoring results.
FIG. 7 shows GVH intensity detection results.
FIG. 8 shows comparison of in vivo anti-tumor detection results between 19 CAR, DU-19 CAR and CU-19 CAR.
FIG. 9 shows comparison of in vivo anti-tumor detection results between 19 CAR and 44-19 CAR.
FIG. 10 shows comparison of in vivo anti-tumor detection results between DU-44-19CAR and 19 CAR.
FIG. 11 shows CAR positive rate detection results.
FIG. 12 shows in vitro pharmacodynamic detection results.
FIG. 13 shows flow cytometer detection results.
FIG. 14 shows exhaustion marker detection results.
FIG. 15 shows GVH intensity detection results.
FIG. 16 shows flow detection results.
FIG. 17 shows flow detection results.
FIG. 18 shows in vitro pharmacodynamic detection results.
FIG. 19 shows factor detection results.
FIG. 20 shows proliferation and vitality detection results.
FIG. 21 shows phenotype detection results.
FIG. 22 shows exhaustion marker detection results.
FIG. 23 shows activation marker detection results.
FIG. 24 shows flow cytometer detection results.
FIG. 25 shows in vitro pharmacodynamic evaluation results.
FIG. 26 shows phenotype detection results.
FIG. 27 shows exhaustion marker detection results.
FIG. 28 shows proliferation and vitality detection results.
FIG. 29 shows GVH intensity detection results.
FIG. 30 shows in vivo efficacy detection results.
FIG. 31 shows results of CAR-T copy numbers in blood of RT-PCR mice.
FIG. 32 is a diagram of gene knockout efficiency.
FIG. 33 is an in vitro killing diagram of DU-19 CAR, CD4 single-negative 4N-19CAR and CD8 single-negative 8N-19CAR.
FIG. 34 shows GVH intensity detection of mixed cells of CD4 single-negative cells and CD8 single-negative cells mixed in the same proportion.

### DETAILED DESCRIPTIONS OF THE EMBODIMENTS

The present disclosure discloses a preparation technique for a universal CAR-T cell, and an use thereof, and those skilled in the art can implement the technique and the use by properly improving process parameters with reference to contents herein. It needs to be specially mentioned that all similar substitutions and modifications will be readily apparent to those skilled in the art, and are considered to be included in the present disclosure. The method and use of the present disclosure have been described through preferred embodiments, and relevant personnel can obviously modify or appropriately change and combine the method and application described herein without departing from the content, spirit and scope of the present invention, so as to realize and apply the technology of the present invention.

Double-negative T cells protected by the present disclosure can be combined with a chimeric antigen receptor (CAR) having any target and structure to obtain technical effects disclosed in the present disclosure, a CD19-targeted chimeric antigen receptor will be taken as an example for detailed description herein. Similarly, vectors stated herein can be any one of a lentiviral expression vector, a retroviral expression vector, an adenovirus expression vector, an adeno-associated virus expression vector, a DNA vector, an RNA vector, and a plasmid. The lentiviral vector is taken as an example for description, and the preparation method can adopt electroporation or non-electroporation, and in this embodiment, the electroporation is taken as an example for description.

In the present disclosure, magnetic bead sorting is performed by using biotin-labeled CD4 and CD8 antibodies coupled with anti-biotin microbeads, or by using CD4 and CD8-positive magnetic beads for column sorting. Further, sorting can be performed by using a flow sorting apparatus or other means capable of obtaining desired fractions, and the desired fractions can be selected finally. Antibodies used to detect knockout efficiency and sorting purity by flow cytometry include: CD3:PE-CY7, CD4:FITC, and CD8:BV510. In the present disclosure, a preparation method for double-negative CAR-T cells includes the following steps: (1) obtaining CD4 and/or CD8 single-positive T cells, and infecting the T cells a virus containing CAR and/or CD44 and/or CD62L structures after activation for a plurality of hours or without activation; and performing RNP electroporation of the T cells infected with the CAR structure after culture for a plurality of hours, and continuing to culture the T cells and sort out target cells; (2) obtaining CD4 and/or CD8 single-positive T cells, performing electroporation of RNP of the T cells after activation for a plurality of hours or without activation, and infecting the T cells a virus containing CAR and/or CD44 and/or CD62L structures after culture for a plurality of hours; and continuing to culture and sort out target cells; (3) obtaining frozen or fresh monocytes, performing electroporation of RNP-nucleated cellss after activation for a plurality of hours or without activation, performing sorting to obtain CD4 and/or CD8 single-positive T cells, performing electroporation of RNP-nucleated cells again, and performing sorting to obtain CD4 and CD8 double-negative T cells; and (4) obtaining frozen or fresh monocytes, performing electroporation of RNP-nucleated cells after activation for a plurality of hours or without activation, performing sorting to obtain CD4 and CD8 double-negative T cells, infecting the T cells with a virus containing CAR and/or CD44 and/or CD62L structures, and continuing to culture to obtain target cells.

The structure of the CAR used in the present disclosure is as follows: an extracellular recognition region (anti-CD19 ScFv) - CD8-derived hinge - CD8-derived transmembrane - intracellular signal (CD137-CD3), with an amino acid sequence shown as SEQ ID NO: 12. DIQMTQSPSSLSASVGDRVTITCRASQDISKYLNWYQQKPGKAPRLLIYHTSRLHSGVPS RFSGSGSGTDYTLTISSLQPEDFATYYCQQGNTLPYTFGGGTRLEIKGSTSGSGKPGSGEG STKGQVQLQESGPGLVKPSQTLSLTCTVSGVSLPDYGVSWIRQPPGKALEWLGVIWGSE TTYYNSSLKTRLTISKDNSKNQVVLTMTNMDPVDTATYYCAKHYYYGGSYAMDYWGQ GSSVTVSSLETTTPAPRPPTPAPTIASQPLSLRPEACRPAAGGAVHTRGLDFACDIYIWAPL AGTCGVLLLSLVITLYCKRGRKKLLYIFKQPFMRPVQTTQEEDGCSCRFPEEEEGGCELR VKFSRSADAPAYKQGQNQLYNELNLGRREEYDVLDKRRGRDPEMGGKPRRKNPQEGLY NELQKDKMAEAYSEIGMKGERRRGKGHDGLYQGLSTATKDTYDALHMQALPPR_{∘}

All raw materials and reagents in the preparation technique for a universal CAR-T cell, and the use of a universal CAR-T cell in the present disclosure are commercially available. The present disclosure will be further described below in conjunction with the examples:

The present disclosure will be further described below in conjunction with the examples:

### Example 1. Results of CD4 Gene and CD8 Gene Knockout in T Cells

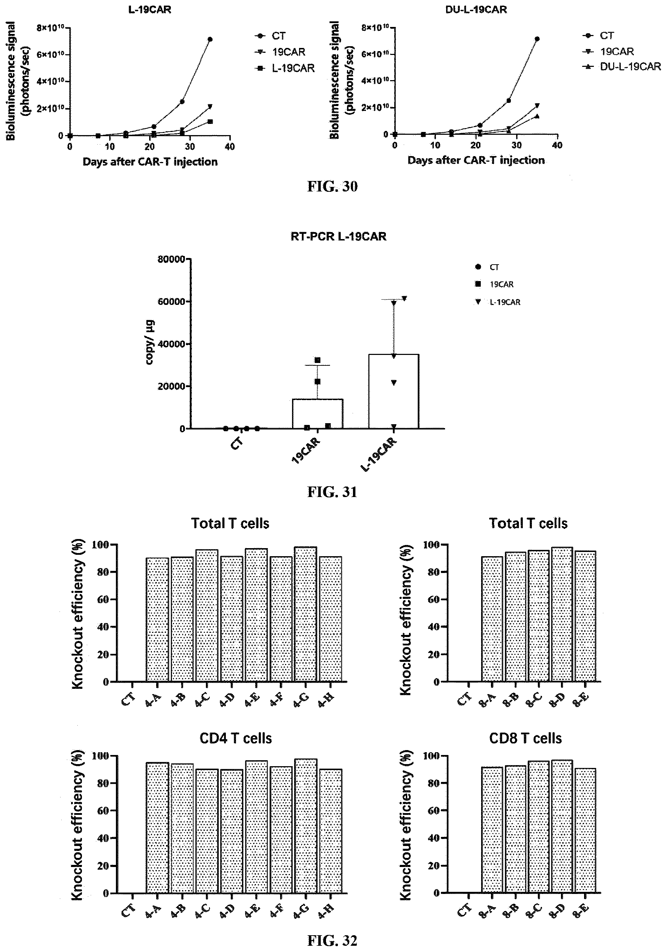

A Lonza electroporation instrument was used, and an electroporation program suitable for performing electroporation of T cells was selected. The molar ratio of Cas9 protein : sgRNA was 1:2 or other suitable ratios, and RNP was incubated in vitro for 5-15 minutes, and was uniformly mixed with an appropriate amount of T cells resuspended in an electroporation buffer and transferred into an electroporation cup for electroporation; after the electroporation, the mixture was transferred into a culture flask containing an appropriate amount of culture medium for continuous culture. After electroporation of 72 h, flow antibodies with detectable fluorescence labels for CD4 or CD8 could be incubated, and knockout efficiency detection was performed. FIG. 32 was a display of partial knockout efficiency among targets shown in Table 6, other targets may also produce relatively good or better knockout effects under different conditions, and any one of target sequences that could cause deletion of gene itself in gene regions of CD4 and CD8 were knocked out.

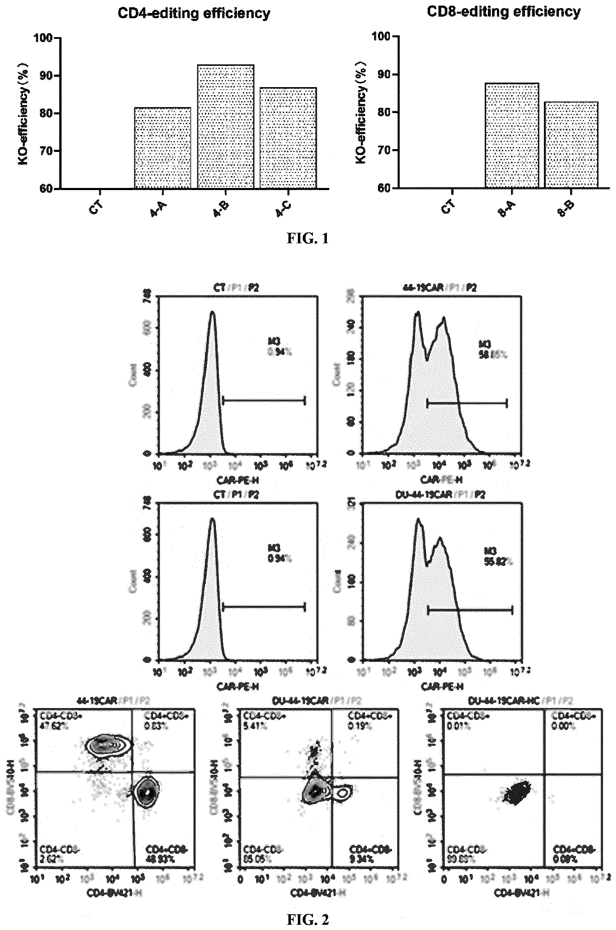

In the present disclosure, target locations for gene editing are shown in Table 1, target sequences in any region in Table 3-Table 5 that could cause deletion of gene itself are subjected to gene editing, and results are shown in FIG. 1. Some of the target sequences used may also be shown as SEQ ID NO 14-33, the sequences are not limited to the sequences shown herein, but can include the target sequences that could cause deletion of gene itself in the gene regions where CD4 and CD8 are knocked out, and include CD4 gene on human chromosome 12: 6,789,528-6,820,799 forward strand, CD8Agene on human chromosome 2: 86,784, 610-86,808,396 reverse strand, and CD8B gene on human chromosome 2: 86,815,339-86,861,924 reverse strand; and target locations for gene editing are shown in Tables 6 and 7, and target sequences in any region below that could cause deletion of gene itself are shown.

**Table 3**

| CD4 genetic map information | | Chromosome location-Chromosome 12 | | |
|---|---|---|---|---|
| Exon No. | Exon/intron | Starting position | Ending position | Length (bp) |
| | 5' upstream sequence | | | |
| 1 | ENSE00002299768 | 6,789,528 | 6,789,662 | 135 |
| | Intron1-2 | 6,789,663 | 6,800,071 | 10,409 |
| 2 | ENSE00003580321 | 6,800,072 | 6,800,187 | 116 |
| | Intron2-3 | 6,800,188 | 6,800,306 | 119 |
| 3 | ENSE00003508863 | 6,800,307 | 6,800,471 | 165 |
| | Intron3-4 | 6,800,472 | 6,814,141 | 13,670 |
| 4 | ENSE00003497892 | 6,814,142 | 6,814,300 | 159 |
| | Intron4-5 | 6,814,301 | 6,814,758 | 458 |
| 5 | ENSE00003569784 | 6,814,759 | 6,814,992 | 234 |
| | Intron5-6 | 6,814,993 | 6,816,055 | 1,063 |
| 6 | ENSE00000716048 | 6,816,056 | 6,816,403 | 348 |
| | Intron6-7 | 6,816,404 | 6,817,129 | 726 |
| 7 | ENSE00003595399 | 6,817,130 | 6,817,330 | 201 |
| | Intron7-8 | 6,817,331 | 6,818,420 | 1,090 |
| 8 | ENSE00003629149 | 6,818,421 | 6,818,542 | 122 |
| | Intron8-9 | 6,818,543 | 6,818,846 | 304 |
| 9 | ENSE00003477403 | 6,818,847 | 6,818,914 | 68 |
| | Intron9-10 | 6,818,915 | 6,819,298 | 384 |
| 10 | ENSE00001182623 | 6,819,299 | 6,820,799 | 1,501 |
| | 3' downstream sequence | | | |

**Table 4**

| CD8A genetic map information | | Chromosome location-Chromosome 2 | | |
|---|---|---|---|---|
| Exon No. | Exon/intron | Starting position | Ending position | Length (bp) |
| | 5' upstream sequence | | | |
| 1 | ENSE0000 1897263 | 86,790,913 | 86,790,777 | 137 |
| | Intron1-2 | 86,790,776 | 86,790,682 | 95 |
| 2 | ENSE00001220182 | 86,790,681 | 86,790,328 | 354 |
| | Intron2-3 | 86,790,327 | 86,789,751 | 577 |
| 3 | ENSE00001011446 | 86,789,750 | 86,789,640 | 111 |
| | Intron3-4 | 86,789,639 | 86,789,434 | 206 |
| 4 | ENSE00001011450 | 86,789,433 | 86,789,323 | 111 |
| | Intron4-5 | 86,789,322 | 86,788,561 | 762 |
| 5 | ENSE00001011445 | 86,788,560 | 86,788,530 | 31 |
| | Intron5-6 | 86,788,529 | 86,785,972 | 2,558 |
| 6 | ENSE00001843887 | 86,785,971 | 86,784,610 | 1,362 |
| | 3' downstream sequence | | | |

**Table 5**

| CD8B genetic map information | | Chromosome location-Chromosome 2 | | |
|---|---|---|---|---|
| Exon No. | Exon/intron | Starting position | Ending position | Length (bp) |
| | 5' upstream sequence | | | |
| 1 | ENSE00001761743 | 86,861,915 | 86,861,823 | 93 |
| | Intron1-2 | 86,861,822 | 86,858,417 | 3,406 |
| 2 | ENSE00002506201 | 86,858,416 | 86,858,057 | 360 |
| | Intron2-3 | 86,858,056 | 86,853,087 | 4,970 |
| 3 | ENSE00002475314 | 86,853,086 | 86,852,997 | 90 |
| | Intron3-4 | 86,852,996 | 86,846,774 | 6,223 |
| 4 | ENSE00002516827 | 86,846,773 | 86,846,684 | 90 |
| | Intron4-5 | 86,846,683 | 86,844,959 | 1,725 |
| 5 | ENSE00002451497 | 86,844,958 | 86,844,922 | 37 |
| | Intron5-6 | 86,844,921 | 86,815,719 | 29,203 |
| 6 | ENSE00001612610 | 86,815,718 | 86,815,557 | 162 |
| | 3' downstream sequence | | | |

**Table 6**

| Sequence ID | Name of sequence | Sequence |
|---|---|---|
| SEOIDNO.14 | sg4-1.1 | gAGGGACTCCCCGGTTCATTG |
| SEOIDNO.15 | sg4-2.1 | gCTGCTGGGAGGAGCGCTAAG |
| SEOIDNO.16 | sg4-3.1 | gTTAAGATTCTTGATGATCAG |
| SEOIDNO.17 | sg4-4.1 | GCACTGAGGGGCTACTACCA |
| SEOIDNO.18 | sg4-5.1 | gCAGCTGGAGCTCCAGGATAG |
| SEOIDNO.19 | sg4-6.1 | gTTTCTTATAGACTATGCTGG |
| SEOIDNO.20 | sg4-7.1 | gTGACGGGCAGTGGCGAGCTG |
| SEOIDNO.21 | sg4-8.1 | gAAAGGTCTCGAAGCGGGAGA |
| SEOIDNO.22 | sg4-9.1 | GGTGGGTCCCCACACCTCAC |
| SEOIDNO.23 | sg4-10.1 | gATTGTGCTGGGGGGCGTCGC |
| SEOIDNO.24 | sg4-11.1 | gCAGGGCCATTGGCTGCACCG |
| SEOIDNO.25 | sg8-1.1 | GCTGCTGTCCAACCCGACGT |
| SEOIDNO.26 | sg8-2.1 | gAGTAGCCCTCGTTCTCTCGG |
| SEOIDNO.27 | sg8-3.1 | gACCCGACGTCGGGCTGCTCG |
| SEOIDNO.28 | sg8-4.1 | GTACTTCAGCCACTTCGTGC |
| SEOIDNO.29 | sg8-5.1 | GTTCTCGGGCAAGAGGTTGG |
| SEOIDNO.30 | sg8-6.1 | gTCGCGGCGCTGGCGTCGTGG |
| SEOIDNO.31 | sg8-7.1 | gAGAGGCGTGCCGGCCAGCGG |
| SEOIDNO.32 | sg8-8.1 | gAGGCGTGCCGGCCAGCGGCG |
| SEOIDNO.33 | sg8-9.1 | GCCCTTGGCCGGGACTTGTG |

**Table 7**

| Gene | Exon No. | Sequence position | Gene | Exon No. | Sequence position |
|---|---|---|---|---|---|
| CD4 | ENSE00003580321 | 1-116 | CD8 | ENSE00001897263 | 1-137 |
| | ENSE00003508863 | 1-165 | | ENSE00001220182 | 1-354 |
| | ENSE00003497892 | 1-159 | | ENSE00001011446 | 1-111 |
| | ENSE00003569784 | 1-234 | | ENSE00001011450 | 1-111 |
| | ENSE00000716048 | 1-348 | | | |
| | ENSE00003595399 | 1-201 | | | |
| | ENSE00003629149 | 1-122 | | | |
| | ENSE00003477403 | 1-68 | | | |

### Example 2. Preparation of DU-19CAR

A method for obtaining DU-19CAR prepared by the technical method of the present disclosure: peripheral blood of a healthy person was taken as a starting sample, from which CD4/CD8 single-positive T cells were respectively sorted out through a magnetic bead technology or a flow cytometry technology. Continuous stimulation was performed using Dynameads containing CD3 and CD28 antibodies to obtain a conventional negative control group CT/a CD4 single positive control group 4P-CT/a CD8 single positive control group 8P-CT; which were infected with CAR-containing lentivirus to obtain a conventional CAR-T/a CD4 single positive 4P-CAR-T/ a CD8 single positive 8P- CAR-T; and

alternatively, an RNP complex of sgRNA and Cas9 protein targeting CD4 and/or CD8 (refer to Example 1 for specific implementation) was subjected to electroporation, flow cytometry or magnetic bead sorting was performed after electroporation to sort out CD4/CD8 double-negative DU-CAR-T and/or CD4/CD8 single-negative 4N-CAR-T/8N-CAR-T cells, and a culture medium added with an appropriate amount of factors, or any other combination of a culture medium and cytokines suitable for culturing T cells, was used as a culture solution. Other steps in the preparation process of CAR-T cells were the same as those in the patents previously filed by the Company.

### Example 3. Evaluation of CD4 and CD8 Double-Negative DU-19CAR

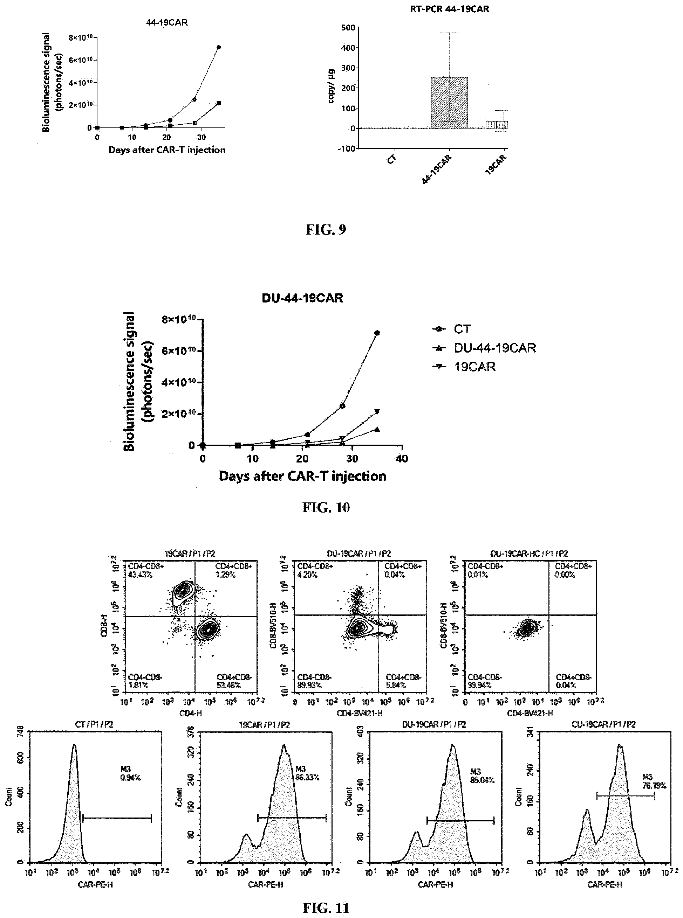

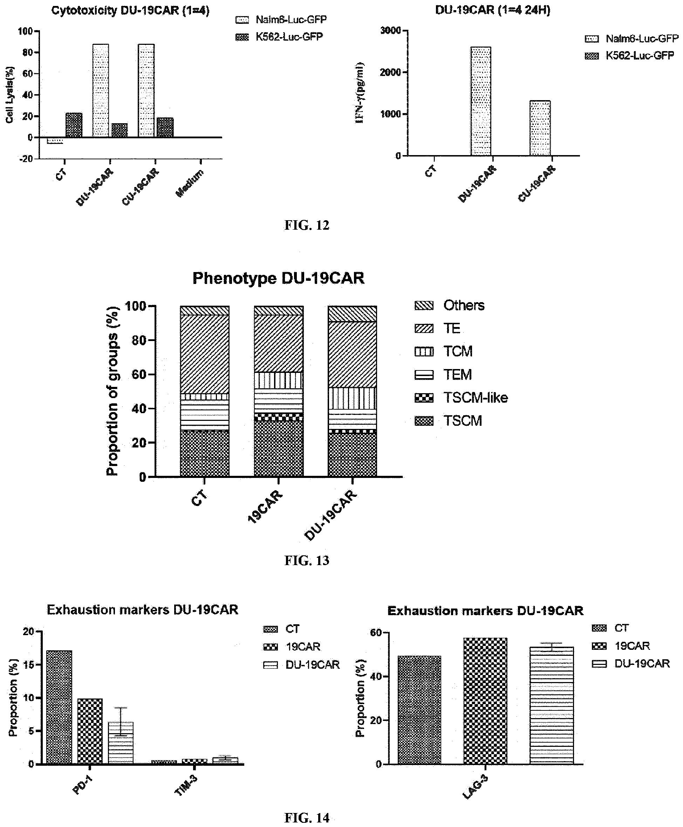

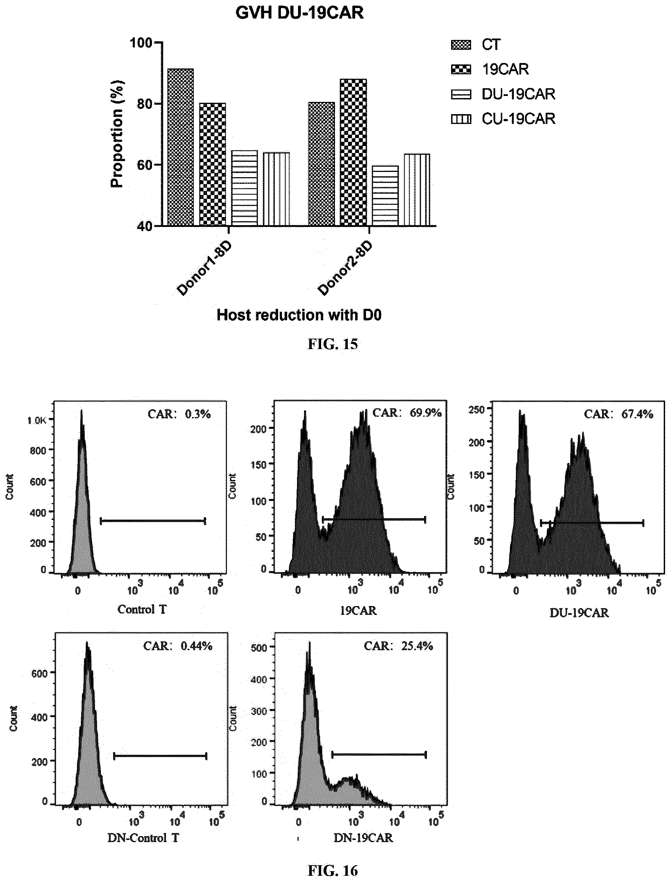

1. CAR positive rate detection method: PE-labeled PL antibody. As shown in FIG. 11, gene editing was performed on CD4 and CD8 on 19CAR, and high-purity double-negative CAR-T cells were sorted out by the magnetic bead. Proportions of CD4 to CD8 in a non-gene editing group were 53.46% and 43.43%, respectively, after gene knockout was performed, a DT ratio was up to 89.93%, and double-negative CAR-T cells having a purity as high as 99.94% were obtained after magnetic bead sorting. Further, the operation did not affect expression of CAR structure on T cells, positive expression levels of DU-19CAR and 19CAR group were both about 85%, while an expression level of conventional universal CU-19CAR was 76%, slightly lower than those of the other two groups.
2. Killer method for in vitro pharmacodynamic evaluation: a fixed effector-target ratiowas used for plating: E: T = CAR-T: tumor cells, cells were cultured in a 1640 complete culture medium for a fixed duration, a luciferase cleavage assay was adopted to detect a survival ratio of target cells, and cytotoxicity efficiency was calculated. A factor secretion level of interferon-γ can be detected with a kit capable of detecting a factor level thereof, and detailed operating procedures were specified in kit instructions of a corresponding manufacturer.
   As shown in the in vitro pharmacodynamic evaluation results in FIG. 12, in vitro killing of the DU-19CAR was equivalent to that of the conventional universal CU-19CAR. When a ratio was 1: 4, killing time was 24 h, and killing efficiency could reach about 90%; however, a factor secretion level of the novel universal DU-19CAR group provided in the present disclosure was twice that of the conventional universal CU-19CAR group, indicating that the method in the present disclosure improved the ability of tumor killing, and was conducive to more powerful attack on the tumor cells.
3. Phenotypic detection method: Flow cytometer detection was performed; antibodies: CCR7: APC, CD45RA: BV 421, CD45RO: Percp5.5; phenotypic classification standards: stem-cell memory T cells : TSCM (CD45RA+CD45RO-CCR7+), stem cell-like memory T cells : TSCM-Like (CD45RA+CD45RO+CCR7+) , effector memory T: TEM (CD45RA-CD45RO+CCR7-), central memory T: TCM (CD45RA-CD45RO+CCR7+), effector T: TE (CD45RA+CD45RO-CCR7-), and others (100%-TSCM-TSCM-Like-TEM-TCM-TE). As shown in FIG. 13, phenotype detection results show that, compared with the conventional autologous 19CAR, the universal DU-19CAR had a relatively close distribution in each group of typing, the TSCM was slightly low, but the TE was slightly higher, which was conducive to instantaneous killing of the tumor cells.
4. Exhaustion marker detection method: Flow cytometer detection was performed; antibodies: PD-1: APC, LAG-3: BV421, TIM-3: Percp5.5. As shown in FIG. 14, according to expression data results of exhaustion marker detection, neither of the two groups of CARs express TIM-3, but compared with the conventional autologous 19CAR, the universal DU-19CAR provided in the present disclosure showed lower expression of PD-1 and LAG-3, indicating that the universal DU-19CAR provided in the present disclosure had a longer persistence, and was thus conducive to improving the anti-tumor ability.
5. GVH intensity determination method: a microsphere absolute counting method was adopted to measure mixed lymphocyte reaction (MLR), a mixed system of donor cells (CAR-T): recipient cells (PBMC of a healthy human body with different HLA typing from donor cells) at a fixed ratio was prepared,, and was cultured with 1640 basic culture medium for 8 days, absolute cell counts were detected by a flow cytometry on days 0 and 8, respectively, and relative intensity of GVH was calculated according to changes in the number of cells.
   Detection results were shown in FIG. 15, indicating that GVH reaction intensity generated by the double-negative DU-19CAR was equivalent to that of CU-19CAR of the conventional universal CAR-T cells, and both were significantly weaker than that of 19CAR of autologous CAR-T cells, and CT group of the control group, further indicating that the DU-19CAR prepared according to the method in the present disclosure could be used for allogeneic CAR-T immunotherapy, same as that of the conventional universal CAR-T cells.
6. In vivo pharmacodynamic evaluation method: mice were introduced on Day 11, quarantine and other series of detection operations were performed on the mice, and mice were included into a group; target cell tail vein injection of 5e5Nalm6-Luc-GFP was performed on Day 3; random grouping was performed on Day 0, and CAR-T cells were transfused according to a number of effective CAR-T cells of 2e6; and in vivo imaging was then performed to observe tumor clearance in the mice every 7 days.

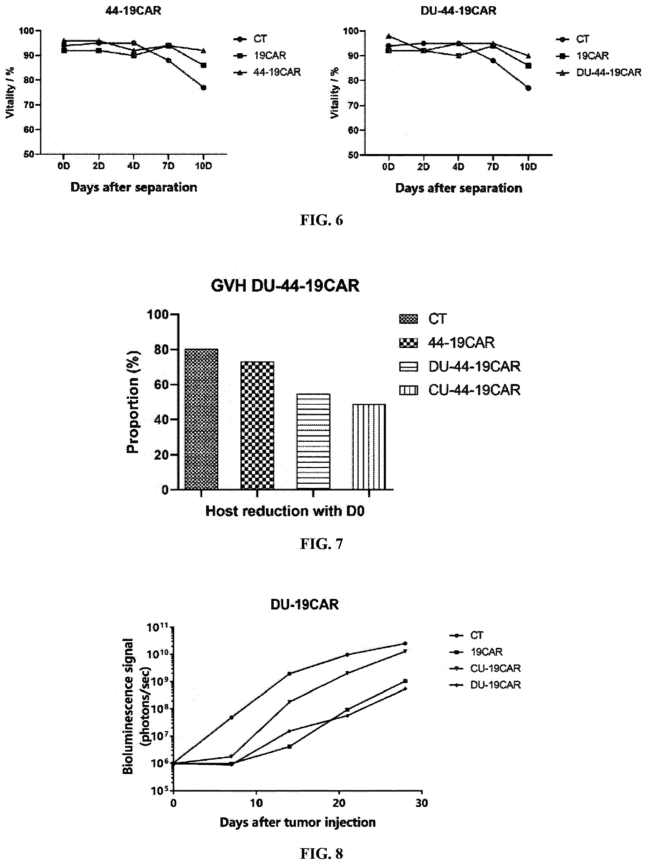

In vivo efficacy results were shown in FIG. 8. Compared with the Control T group, the 19CAR and DU-19CAR groups, and the CU-19CAR group have significant tumor inhibitory effects in vivo. Analysis from fluorescence value curve data of the DU-19CAR group and the CU-19CAR group indicated that the in vivo efficacy of the DU-19CAR group was significantly better than that in CU-19CAR group, but the DU-19CAR provided in the present disclosure had the in vivo efficacy better than that of the autologous group 19CAR, indicating that anti-tumor effects of the novel universal CAR-T cells provided in the present disclosure were not only far better than those of the conventional universal CAR-T cells, but also better than those of the conventional autologous CAR-T cells, which was also one of expected achievements of the present disclosure.

In summary, in vitro and in vivo pharmacodynamic evaluation, phenotypic detection, exhaustion marker detection were performed on the DU-19CAR provided in the present disclosure, evaluation results indicate that the DU-19CAR was superior to the conventional universal CAR-T cells in all aspects; and a microsphere absolute counting method was adopted to measure mixed lymphocyte reaction (MLR), the results showed that GVH reaction intensity generated by the novel DU-19CAR provided in the present disclosure was equivalent to that of the conventional universal CAR-T cells (CU-19CAR), and could be used for allogeneic transfusion therapy.

### Example 4. Evaluation of DU-19CAR Mixed by CD4 Single-Negative, CD8 Single-Negative and CD4 Single-Negative, and CD8 Single-Negative

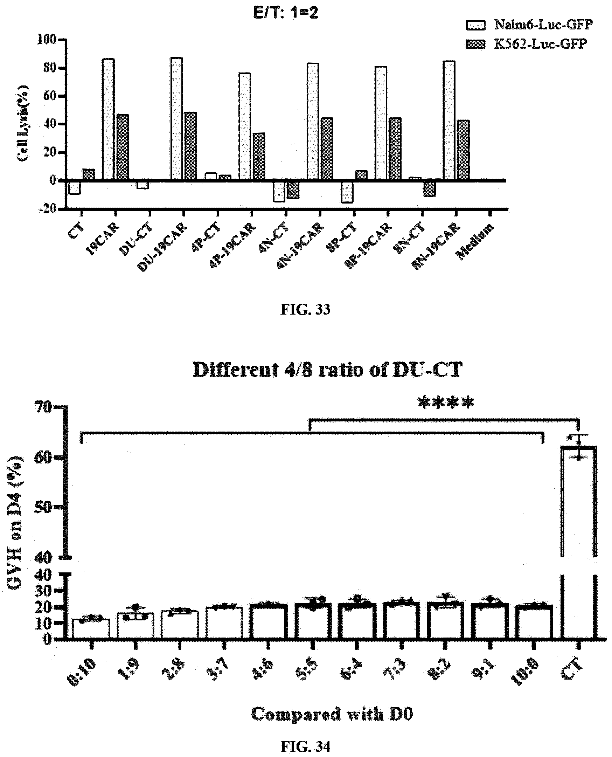

1. In vitro pharmacodynamic evaluation: As shown in the in vitro pharmacodynamic evaluation results in FIG. 33, the in vitro anti-tumor capabilities of DU-19CAR, CD4 single-negative 4N-19CAR and CD8 single-negative 8N-19CAR are equivalent to those of the control group autologous 19CAR, and killing efficiency thereof can reach above 80%. Non-specific killing shown in K562 -Luc-GFP is a normal phenomenon, indicating that the in vitro anti-tumor capability of the DU-19CAR provided in the present disclosure or the DU-19CAR composed of CD4-negative and CD8-negative 19CAR is not attenuated due to gene editing.
2. GVH Intensity Determination: Mixed lymphocyte reaction (MI,R) was performed, flow detection was made, and a fixed ratio was configured: firstly, recipient cells (PBMC of a healthy human with different HLA typing from donor cells) were treated with Mitomycin C to inhibit their growth, interference was eliminated, donor cells and/or recipient cells were dyed with different color dyes (CTV and/or CFSE) to distinguish, and the donor cells (activated T cells) to the recipient cells were mixed at a fixed ratio, where the donor cells were DU-CT and CT composed of CD4-positive/negative and CD8-positive/negative T cells, and the composition ratio of each group of cells was shown in Table 8. After the mixing was completed, flow detection was performed immediately to distinguish respective proportions thereof, a 1640 basic culture medium was used for culture, and changes in the respective proportions were detected by the flow detection at fixed time points, and relative GVH intensity was calculated according to changes in cell proportions.

After 4 days of culture as shown in FIG. 34, the GVH Intensity of the DU-CT group is significantly lower than the control group CT which could be seen from a cell proliferation ratio caused by rejection of the donor cells to the host PBMC.These results indicate that the double-negative CAR-T cells prepared according to the method in the present disclosure can be used for allogeneic CAR-T immunotherapy.

**Table 8**

| Grouping name | 4N-CT (%) | 8N-CT (%) | 4P-CT (%) | 8P-CT (%) | PBMC (%) |
|---|---|---|---|---|---|
| 0:10 | 0 | 50 | 0 | 0 | 50 |
| 1:9 | 5 | 45 | 0 | 0 | 50 |
| 2:8 | 10 | 40 | 0 | 0 | 50 |
| 3:7 | 15 | 35 | 0 | 0 | 50 |
| 4:6 | 20 | 30 | 0 | 0 | 50 |
| 5:5 | 25 | 25 | 0 | 0 | 50 |
| 6:4 | 30 | 20 | 0 | 0 | 50 |
| 7:3 | 35 | 15 | 0 | 0 | 50 |
| 8:2 | 40 | 10 | 0 | 0 | 50 |
| 9:1 | 45 | 5 | 0 | 0 | 50 |
| 10:0 | 50 | 0 | 0 | 0 | 50 |
| CT | 0 | 0 | 50 | | 50 |

### Example 5: Results of Comparison between Natural DNT or DN-CAR-T (DN-19CAR: Native CD4⁻CD8⁻T cells) Prepared Therefrom and Universal DU-CAR-T (DU-19CAR: CD4⁻CD8⁻T cells Obtained Through Gene Editing) Prepared by the Technique in the Present Disclosure

Natural DNT refers to natural double-negative T cells in peripheral blood of a human body that do not express CD4 and CD8, and this cell antigen presentation pathway is not restricted by MHC and has an extremely low probability of isotype rejection in allogeneic use (accounting for 1-5% of total lymphocytes in the peripheral blood), but the proportion of these cells in the peripheral blood of the human body is extremely low, featuring high difficulty in in-vitro amplification, and therapeutic effects on indications are not very good. The universal double-negative cells prepared by the technique in the present disclosure are initially sourced from CD4 single-positive or CD8 single-positive T cells (accounting for 40-80% of total lymphocytes in the peripheral blood) in the peripheral blood of the human body, antigen presentation process of the cells depends on MHC restriction, and cannot be directly used for allogeneic use, therefore, further operation needs to be performed to avoid the isotype rejection. The present disclosure makes the CD4 and CD8 inactivated through the gene editing technology, so that the CD4⁻ CD8⁻ double-negative T cells are obtained. The present disclosure proves that this type of cells has necessary conditions applicable to allogeneic therapy, same as the conventional universal T cells, refer to GHV-related data.
1. Method for obtaining natural DNT: starting sample: peripheral blood of a healthy person. (1) CD4/CD8 SPT cells (CD4 or CD8 cells) were removed through magnetic beads, continuously stimulated with an antibody containing CD3 and CD28 for 24-48 h, and then cultured with a culture medium containing IL2 and IL4 (and/or) IL7 (and/or) IL15 (and/or) IL21 for enrichment culture. (2) CD3⁺CD4⁻/CD8⁻T cells were sorted out through a flow sorting method, continuously stimulated with an antibody containing CD3 and CD28 for 24-48 h, and then cultured with a culture medium containing IL2 and IL4 (and/or) IL7 (and/or) IL15 (and/or) IL21 for enrichment culture.

Method for obtaining DN-19CAR: obtaining natural DNT, and culture for 24 h to be infected with a lentivirus containing CAR.

6 batches of peripheral blood from healthy people from different sources were collected, and PMBC thereof was sorted out, 3 batches with the highest DNT contents were screened through testing, with screening indicators: CD3⁺CD4⁻CD8⁻, initial cells for preparing DU-19CAR were SPT, with screening indicators: CD3⁺, CD4⁺/CD8⁺, and results were shown in Table 9.

**Table 9**

| Donor No. | 555 | 556 | 557 | 558 | 559 | 560 |
|---|---|---|---|---|---|---|
| DNT percentage (%) | 4.97 | 2.9 | 3.19 | 13.8 | 8.07 | 16.85 |
| SPT percentage (%) | 94.82 | 96.67 | 96.38 | 85.79 | 91.56 | 82.68 |

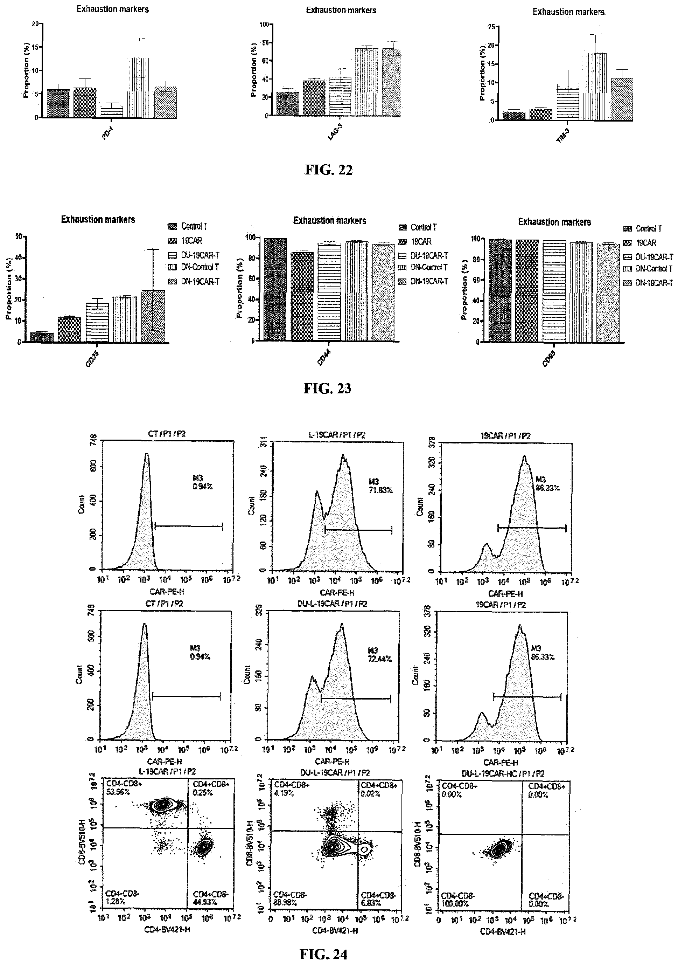

Calculation was made by taking le8 as a starting count, and statistics on the number of CAR-T cells obtained on Day 6 were shown in Table 10. As could be seen from data in the table, with the same starting count of PBMC, the number of DU-19CAR cells obtained at least 3 times the DN-19CAR cells, and up to 35 times or more at maximum, after batches with a higher proportion of DNT were screened. As shown in FIG. 23, proliferation data monitored starting from Day 6 of activation indicated that the DN-19CAR cells was far inferior to the DU-19CAR cells in a later stage of proliferation. It could be seen that the DU-19CAR cells had a wider range of use, and could meet the needs of various uses, especially in the field of clinical use that required higher counts of cells. As could be seen from FIG. 16 that a CAR positive rate of the DU-CAR-T cells is greater than 60%, which is much higher than 25.4% of that of the N-DN-CAR-T cells, that is, it presents a higher proportion of effective cells and becomes more conducive to killing tumor cells.

**Table 10**

| Cell type | Starting count of PBMC | Effective cell counts obtained on Day 6 | | |
|---|---|---|---|---|
| | | Donor558 | Donor559 | Donor560 |
| DU-19CAR | le8 | 8.8057E7 | 7.0018E7 | 8.8057E7 |
| DN-19CAR | le8 | 2.43E7 | 2E6 | 2.43E7 |

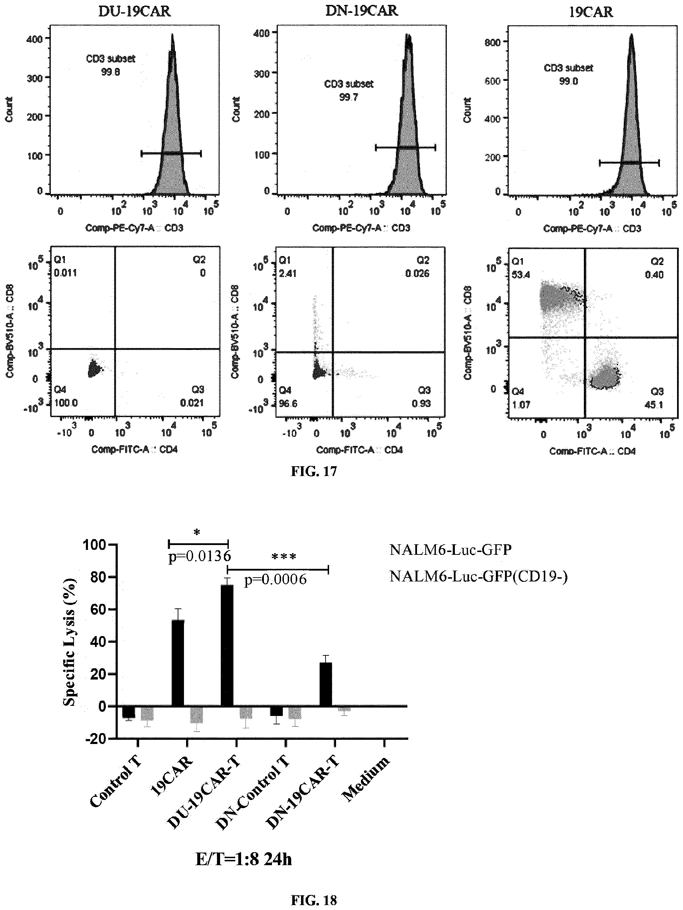

3. Method for obtaining DU-19CAR: Preparation method was the same as described above, magnetic bead sorting was performed by using biotin-labeled CD4 and CD8 antibodies, and coupled with anti-biotin microbeads for column sorting, as shown in FIG. 17, and negative fractions were then selected. Antibodies for flow detection knockout efficiency and sorting purity include: CD3: PE-CY7, CD4: FITC, and CD8: BV510.

As can be seen from FIG. 17, a purity ratio of the DU-19CAR cells was as high as 100%, proving a high sorting purity; a DNT ratio of the DN-CAR-T cells was 96.6%, proving that the DNT obtained by an enrichment method was higher, but slightly lower than the method in the present disclosure, further indicating that the DU-CAR-T cells obtained by the method in the present disclosure were more advantageous in terms of the safety.

4. In vitro pharmacodynamic evaluation, as shown in FIG. 18, when an effector-target ratio was 1: 8, killing results showed that the DU-19CAR in the present disclosure was statistically different from the conventional autologous 19CAR, and the DU-19CAR in the present disclosure was significantly statistically different from the DN-19CAR prepared from the natural DNT, demonstrating that killing ability of the DU-CAR-T cells in the present disclosure was significantly stronger than that of the DN-CAR-T cells, and was slightly better than that of the autologous CAR-T cell groups.

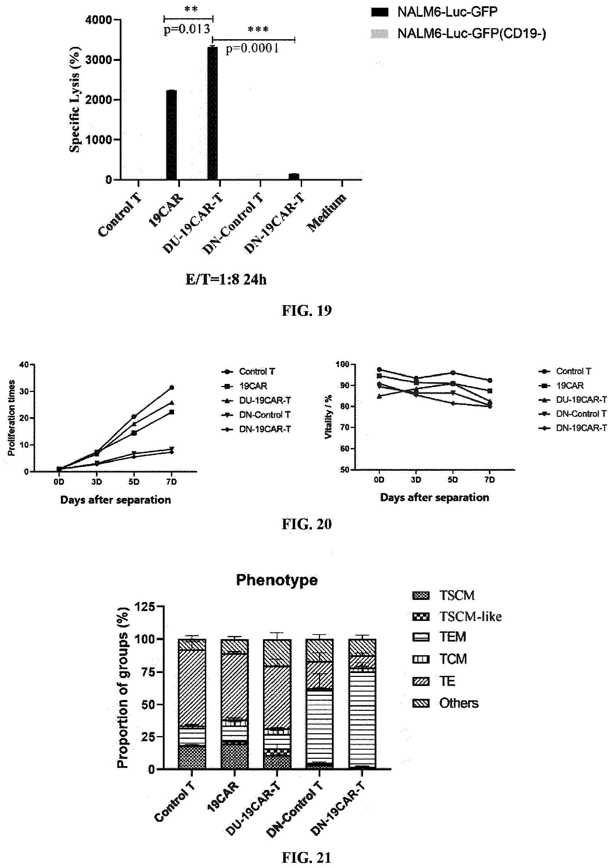

5. Factor detection results are shown in FIG. 19, detection was performed by any kit capable of detecting interferon-γ, a supernatant culture medium after killing was detected, and the detection method was subject to the instructions of corresponding reagent. Results indicate that the DU-19CAR in the present disclosure was statistically different from the conventional autologous 19CAR, and was significantly different from the DN-19CAR prepared from the natural DNT. This reflected stronger in vitro efficacy of the double-negative universal CAR-T cells in the present disclosure from the level of factor secretion.

6. Proliferation and vitality monitoring: High-purity double-negative DU-19CAR (Day 5 or Day 6 after activation) was sorted out through magnetic beads to perform monitoring. Monitoring method: A fixed starting count of cells (such as le6) was taken from each group for monitoring, monitoring was performed every 2-3 days until a group of cells stopped proliferation, and vitality thereof began to fall, and cells could not be discarded throughout the monitoring process. As shown in FIG. 20, proliferation ability and cell viability of the universal DU-CAR-T in the present disclosure were significantly better than those of natural double-negative DN-Control T cells or double-negative DN-19CAR cells prepared therefrom, indicating that proliferation and viability of the universal DU-19CAR cells obtained according to the preparation method in the present disclosure were not affected, and even having supervising effects. However, the natural DNT was of poor proliferation, and was difficult to quickly meet the therapeutic demand, moreover, the production cost was also greatly increased, suggesting difficulty in clinical translation and utilization.

7. Phenotype, exhaustion marker, activation marker detections:
1) phenotype detection results were shown in FIG. 21, a proportion of TE in the DU-19CAR group prepared according to the present disclosure was obviously higher than that of the DN-19CAR group. Since action duration of the CAR-T was short, and instantaneous killing ability was an important indicator of drug efficacy, the killing results indicated that the DU-19CAR prepared according to the present disclosure had stronger killing ability.
2) Exhaustion marker detection results were shown in FIG. 22, expression levels of the three exhaustion molecules in the DU-19CAR of the present disclosure were lower than that of the DN-19CAR, indicating that the DU-19CAR of the present disclosure featured stronger persistence and slower apoptosis speed, thereby exerting better anti-tumor effects.
3) Activation marker detection method: Flow cytometer detection was performed; antibodies: CD25: FITC, CD44: BV421, and CD95: BV4711. Activation marker detection results were shown in FIG. 23, indicating that CD95 and CD44 both exhibited similar expressions, except that CD25 showed slight difference.

### Example 6. Example 6 Preparation of T Cells and 44-19CAR (Chimeric Antigen Receptor T Cells) Cells Expressing CD44

CD44 can be combined with a chimeric antigen receptor (CAR) having any target and structure to obtain technical effects disclosed in the present disclosure, a CD19-targeted chimeric antigen receptor will be taken as an example for detailed description herein. Similarly, vectors stated herein can be any one of a lentiviral expression vector, a retroviral expression vector, an adenovirus expression vector, an adeno-associated virus expression vector, a DNA vector, an RNA vector, and a plasmid. The lentiviral vector is taken as an example for description herein.

### 1) Construction of a vector expressing CD44

A gene fragment corresponding to the CD44 amino acid sequence was shown in SEQ ID NO 12, the abovementioned target fragment and the vector fragment were connected through T4 ligase (purchased from Promega Company) to obtain a lentiviral vector expressing the chimeric antigen receptor, and plasmids were extracted through a plasmid extraction kit (purchased from Invitrogen Company). Specific method was stated in the instructions thereof. Vectors stated herein can be any one of a lentiviral expression vector, a retroviral expression vector, an adenovirus expression vector, an adeno-associated virus expression vector, a DNA vector, an RNA vector, and a plasmid. In order to illustrate specific preparation solutions, the lentiviral vector was taken as an example for description herein:

The gene sequence of CD44 molecule obtained therefrom was subjected to enzymatic cleavage with restriction endonucleases Nhel and Xhol (purchased from Thermo Company), pRRLSIN.cPPT.PGK-GFP.WPRE plasmid (http://www.addgene.org/12252/) of a lentiviral expression vector was also subjected to enzymatic cleavage with restriction endonucleases Nhel and Xhol, and enzymatic cleavage reaction was performed according to the instructions thereof. Enzymatic cleavage products were sorted out by agarose gel electrophoresis, a DNA fragment thereof was recovered by using an agarose gel DNA fragment recovery kit, a synthesized CD44 sequence fragment with Nhel and Xhol enzymatic cleavage sites was subjected to enzymatic cleavage with restriction endonucleases Nhel and Xhol, and the products obtained were then ligated to DNA fragment recovered above, such that the vector capable of expressing CD44 was obtained.

### 2) Construction of a vector expressing CD44 and CAR genes capable of specifically recognizing tumors

A chimeric antigen receptor sequence containing a single-chain antibody ScFv with an anti-human CD19 antigen, a hinge region, a transmembrane region, and an intracellular signal segment was synthesized.

PCR amplification was performed using the chimeric antigen receptor sequence and the CD44 molecule as templates respectively, and a reaction system was loaded according to the instructions of KOD FX NEO DNA polymerase (purchased from TOYOBO Company), PCR reaction conditions were: pre-denaturation at 95°C for 5 min, denaturation at 95°C for 10 s, annealing at 55°C for 15 s, extension at 68°C for 30 s, with 30 cycles. Amplified products were identified with 1% agarose (w/v) gel. DNA fragment was then recovered by using a recovery kit (Promega Company), and specific method was stated in the instructions thereof. A chimeric antigen receptor and a CD44 molecular fragment were recovered and obtained, and the DNA recovered fragment was sent for sequencing.

### (2) Construction of a lentiviral vector expressing chimeric antigen receptor: Targeting CD19CAR-linker peptide (polycistronic structure) - CD44

The above CAR molecule was linked to the CD44 molecule using a polycistronic structure, the polycistronic structure was a self-cleaving polypeptide or an internal ribosome entry site (IRES), and the self-cleaving polypeptide was T2A, P2A, E2A or F2A. P2A was taken as an example herein to obtain an expression vector targeting CD19CAR-P2A-CD44.

Enzymatic cleavage reaction was performed according to the instructions. Enzymatic cleavage products were sorted out by agarose gel electrophoresis, a DNA fragments thereof was recovered by using an agarose gel DNA fragment recovery kit, and the target fragment and the vector fragment were connected through T4 ligase (purchased from Promega Company) to obtain a lentiviral vector expressing the chimeric antigen receptor and CD44 molecules. 5 µl of the lentiviral vector was taken to transform E coli TOP10, which was cultured at 30°C for 16 h, a single clone was then selected, the selected single clone was cultured at 30°C for 12 h, and plasmids thereof were extracted through a plasmid extraction kit (purchased from Invitrogen Company). Specific method was stated in the instructions thereof. The extracted plasmids were identified by restriction endonuclease HindIII enzymatic cleavage electrophoresis.

### 3) Preparation of a lentivirus by using the vectors obtained in the steps 2) and 3)

### Packaging of lentivirus

In this example, lentivirus packaging was performed using a calcium phosphate method, with specific steps as follows: a DMEM medium containing 10% FBS (w/v) was used to culture 293T cells to a better state; the 293T cells were transferred at a density of 1 × 105/cm² to one culture flask with a capacity of 75 cm² and cultured for 22 h, to ensure that a cell confluence was 70 -80% during transfection; the solution was replaced with a preheated DMEM medium containing 2% FBS (w/v), and the cells were cultured for 2 h for later use; 680 µL of ddH₂O, 20 µg of lentiviral vector (the vectors described in the steps 1) and 2)), and 100 µL of 2.5 mM of CaCl₂ were added in a 15 mL centrifuge tube and mixed uniformly to obtain a mixed solution, 2 × HBS was added dropwise to the mixed solution by using a pipette, the mixed solution was then blown and mixed uniformly by using a 10 mL pipettor, and then stood at room temperature for 15 min, the mixed solution was then added dropwise to the cells prepared above, the cells were continuously cultured for 12-16 h, and the culture medium was replaced with a DMEM medium containing 10% FBS (w/v). After the cells were cultured for 48 h and 72 h, the cell supernatant was collected for virus purification.

### (2) Purification of lentivirus

The virus supernatant was collected in a 50 mL centrifuge tube, centrifuged at 3000 r/min for 10 min, and then filtered with a 0.45 µm filter membrane, and filtrate was obtainded and centrifuged at 3000r/min for 10min and then transfer to a new 50mL centrifuge tube. 50% PEG 6000 (w/v) and 4M NaCl were added to obtain a mixed solution according to an amount of the virus supernatant, the mixed solution was brought to a total volume of 35 mL with medical saline (a final concentration of PEG6000 was 8.5%, and a final concentration of NaCl was 0.34M), and then stood in a 4°C refrigerator for 90 min, and the mixed solution was mixed uniformly by turning upside down every 30 min to obtain a sample; and the sample was centrifuged at 4°C and 5000 r/min for 30 min, the supernatant thereof was discarded, virus was resuspended with 200 µL of a DMEM medium containing 10% FBS and then packed into 1.5 mM EP tubes, with 40µL per tube, and kept at -80°C for later use.

### (3) Determination of lentivirus titer

### A. 293T Cell infected with virus

293T cells were plated into a 24-well plate containing 10% FBS (w/v) DMEM culture medium 12 h in advance to ensure that a cell confluence before virus infection ranged from 40% to 70%, and the cells were in good condition. 1 µL of 6 g of Polybrene solution was added in each well, 1 µL of the purified lentiviral vector was taken and diluted 10 times with medical normal saline to prepare a working solution, the working solution was added in the corresponding well, the culture medium was replaced with a DMEM medium containing 10% FBS (w/v), and then centrifuged at 1000 r/min for 5 min after infection for 72 h to collect cells, and genome was then extracted.

### B. Extraction of genome

The collected cells were resuspended with PBS and centrifuged at 1000 r/min for 5 min, the supernatant was discarded, and operation was repeated once; the cells were resuspended with 200 µL of PBS, protease K was added to obtain a solution, the solution was blown and mixed uniformly using 200 µL of Al lysis buffer, and incubated at 56°C for 10 min; 200 µl of pre-cooled ethanol was added, the solution was mixed uniformly by turning upside down and then transferred to a filter column, stood at room temperature for 1 min, and centrifuged at 8000 r/min for 1 min, and the supernatant thereof was discarded; 700 µl of AW1 solution was then added and centrifuged at 8000 r/min for 1 min, and the supernatant thereof was discarded; 500 µl of AW2 solution was then added and centrifuged at 8000 r/min for 1 min, the supernatant thereof was discarded, the filter column was transferred into a new 1.5 mL EP tube, and stood for 1 min with the cap opening to make ethanol volatilized; and 50µL sterilize ddH₂O was added (preheated at 60°C), and the solution was stood for 2 min and centrifuged at 12,000 r/min for 1 min. (genome extraction kit was QIAamp DNA Blood Mini Kit purchased from Qiagen Company).

### C. qRT -PCR determination of virus titer

A reaction system was as follows: 10µL of SYBR^{®}_Premix Ex Tap^{™} II (2 ×), 1 µL of upstream primer (GAG up), 1 µL of downstream primer (GAG dn), 1 µL of the extracted genome, 7 µL of RNase-Free dH₂O, and at least 3 replicate wells for each sample and standard. Amplification was then performed according to the following procedures: pre-denaturation at 95°C for 5 s, denaturation at 95°C for 5 s, annealing at 60°C for 30 s, extension at 72°C for 30 s, data was analyzed by using analysis software after the reaction was finished, and virus titer was then calculated according to a standard curve, and results were expressed in TU/ml.

### 4) T cells infected with a lentivirus

### (1) Insolation of human peripheral blood mononuclear cells

About 60 mL of peripheral blood of a healthy person was collected by using a blood collection tube added with anticoagulant, the peripheral blood was added in two 50 mL centrifuge tubes, with each tube added with 30 mL, and 7.5 mL of hydroxyethyl starch was added in each tube for dilution. The centrifuge tubes was stood at room temperature for natural sedimentation about 30 min, plasma at an upper layer was collected, centrifuged at 1400 r/min for 15 min, and then resuspended with normal saline for precipitation. Cell suspension was carefully loaded onto a lymphocyte separation medium at a volume ratio of 1: 1, and centrifuged at a gradient of 400g for 15 min, and speed reduction DEC of a centrifuge was set to 5. After centrifugation, the centrifuge tube was divided into four layers from top to bottom, that is, a plasma layer, a whitish ring peripheral blood mononuclear cell (PBMC) layer, a transparent separation liquid layer and an erythrocyte layer. The PBMC of the second layer was carefully sucked and transferred to a new centrifuge tube and washed twice with normal saline, which was centrifuged at 400 for 10 min for a first time, and centrifuged at 1100 r/min for 5 min for a second time, cells were resuspended with normal saline, and PBMC cell suspension was pipetted and transferred to a culture flask, and then cultured with an RPMI 1640 complete medium containing 10% FBS (w/v).

### (2) T lymphocytes infected with a lentiviral vector

A newly-prepared mononuclear cell PBMC was cultured with an RPMI 1640 complete medium containing 10% FBS, and anti-CD3 monoclonal antibody was added for activation on Day 1; lentiviral infection was performed on the first three days, the lentiviral vector stated in the steps 1) and 2) was added at 2 multiplicity of infection (MOI), and the uninfected peripheral blood mononuclear cell (PBMC) was taken as a blank control; and after 24 h, the culture medium with was replaced with an RPMI 1640 complete medium containing 500 IU/ml of recombinant human IL-2, and culture was continued for 10-20 d.

In some embodiments, the T cells co-expressing CD44 and CAR could also be prepared using the following solution:
A. T cells expressing CD44 were prepared according to the solution in the step 1) above, after the T-cells expressing CD44 were obtained, CAR gene was transduced on the T cells expressing CD44 to obtain T cells expressing both CD44 and CAR.
B. T cells expressing CAR (CAR-T cells) were obtained by using the steps similar to those in the step 2) above, the difference was that a vector expressing CAR did not contain CD44 gene, the CD44 gene was further transduced on the basis of obtaining the CAR-T cells, and T cells (44-19CAR) expressing both CD44 and CAR were then obtained.

Expression of CD44 and CAR was shown in FIG. 2, and both CD44 and CAR can be normally expressed in the T cell.

### Example 7. Preparation of DU-44-19CAR

### 1. Gene knockout experiment

DU-T cells with both CD4 and CD8 knocked out were obtained with reference to Example 1.

### 2. Method for obtaining DU-44 -19CAR

Peripheral blood of a healthy donor was taken as a starting sample. CD4/CD8 single-positive T cells were respectively sorted out through a magnetic bead technology or a flow cytometry technology, continuous stimulation was performed using Dynameads containing CD3 and CD28 antibodies, a lentivirus containing CAR was infected, an RNP complex of sgRNA and Cas9 protein targeting CD4 and CD8 was subjected to electroporation, flow cytometry or magnetic bead sorting was performed after electroporation to sort out DU-CAR-T cells, and an culture medium added with an appropriate amount of factors, or any other combination of a culture medium and cytokines suitable for culturing T cells, was used as a culture solution.

Method for obtaining DU-44 -19CAR (either of the following method is acceptable): 1. CD4 and/or CD8 single-positive T cells were obtained, the T cells were infected with a virus containing CD44 and CAR structures after activation for a plurality of hours, RNP electroporation was performed on the T cells infected with the CAR structure after culture for a plurality of hours, and continuous culture was performed to sort out target cells; or 2. CD4 and/or CD8 single-positive T cells were obtained, electroporation of RNP was performed on the T cells after activation for a plurality of hours, the T cells were infected with a virus containing CD44 and CAR structures after culture for a plurality of hours, and continuous culture was performed to sort out target cells.

Preparation order of method for obtaining 44-19CAR: CD4 and/or CD8 single-positive T cells were obtained, the T cells were infected with a virus containing CD44 and CAR structures after activation for a plurality of hours, and continuous culture was performed.

### Example 8. Evaluation of DN-44-19CAR and 44-19CAR

### 1. Expression of CAR

Gene editing was performed on CD4 and CD8 of 44-19CAR cells, high-purity double-negative CAR-T cells were sorted out by the magnetic bead, and the operation did not affect expression of CAR structure on T cells. As can be seen from FIG. 2, expression levels of CAR on 44 -19CAR and DU-44-19CAR cells were both higher than 55%. A proportion of dual-negative CAR-T after DU-19CAR sorting was as high as 99% or more.

### 2. In vitro killing.

A fixed-effect target ratio plate was employed: E: T = CAR-T: tumor cells, cells were cultured in a 1640 complete culture medium for a fixed duration, a luciferase cleavage assay was adopted to detect a survival ratio of target cells, and cytotoxicity efficiency was calculated. A factor secretion level of interferon-γ can be detected with a kit capable of detecting a factor level thereof, and detailed operating procedures were specified in kit instructions of a corresponding manufacturer.

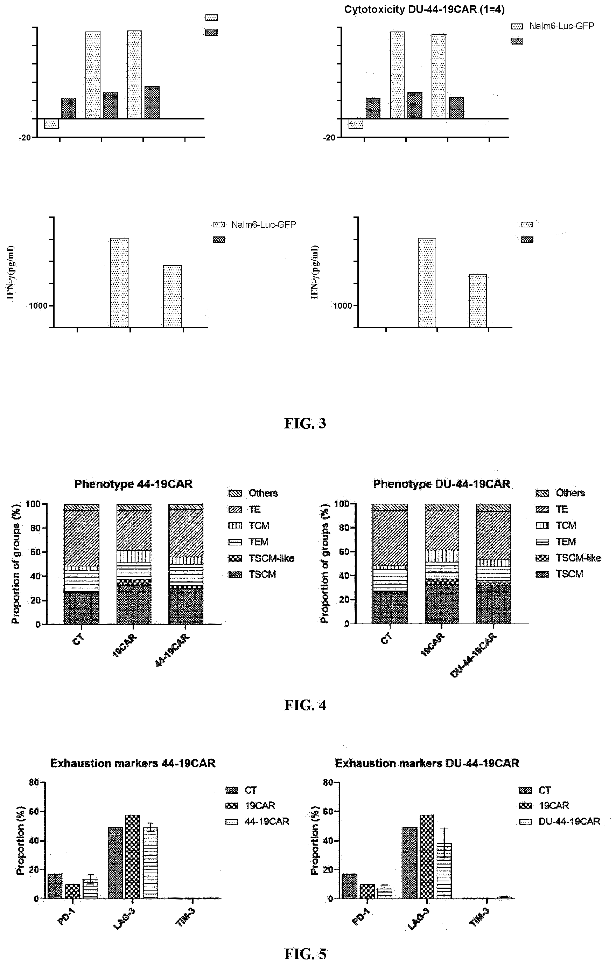

As shown in FIG. 3, Tables 11-14, in vitro pharmacodynamic evaluation results indicated that in vitro efficacy of 44-19CAR was equivalent to that of the autologous 19CAR, when an effector-target ratio was 1:4, and killing time was 24 h, killing efficiency thereof could reach 90% or above; killing ability of the DU-44-19CAR was also equivalent to that of the 19CAR; and results of cytokine secretion of 44-19CAR and DU-44-19CAR were both lower than that of 19CAR, proving that with the same killing ability, 44-19CAR and DU-44-19CAR prepared according to the present disclosure secreted smaller amount of factors, but their killing abilities were not affected, so that a risk of cytokine release syndrome was reduced, and safety of CAR-T therapy was improved. Specifically, IFN-γ, as a TH1-type molecule, was a manifestation of specific function of the CAR-T cells at present. However, on the other hand, excessively high IFN-γ secretion was associated with cytokine release syndrome (CRS for short), side effects generated by CAR-T therapy, therefore, high secretion of lFN-γ had both advantages and disadvantages. Data corresponding to FIG. 3 was as follows:

**Table 11 Killing Data of 44-19CAR**

| | Nalm6-Luc-GFP | K562-Luc-GFP |
|---|---|---|
| CT | -10.63829804 | 22.80829979 |
| 19CAR | 95.12959388 | 29.24838592 |
| 44-19CAR | 96.00232116 | 35.45701876 |
| Medium | 0 | 0 |

**Table 12 Killing Data of DU-44-19CAR**

| | Nalm6-Luc-GFP | K562-Luc-GFP |
|---|---|---|
| CT | -10.63829804 | 22.80829979 |
| 19CAR | 95.12959388 | 29.24838592 |
| DU-44-19CAR | 92.49980665 | 23.73817359 |
| Medium | 0 | 0 |

**Table 13 IFN-γ (pg/ml) Secretion Data of 44-19CAR**

| | Nalm6-Luc-GFP | K562-Luc-GFP |
|---|---|---|
| CT | 0 | 0 |
| 19CAR | 4080 | 0 |
| 44-19CAR | 2840.33 | 0 |

**Table 14 IFN-γ (pg/ml) Secretion Data of DU-44-19CAR**

| | Nalm6-Luc-GFP | K562-Luc-GFP |
|---|---|---|
| CT | 0 | 0 |
| 19CAR | 4080 | 0 |
| DU-44-19CAR | 2444.33 | 0 |

### 3. Phenotypic results and exhaustion marker

Flow cytometer detection was performed; antibodies: CCR7: APC, CD45RA: BV 421, CD45RO: Percp5.5; phenotypic classification standards: T memory stem-cell: TSCM (CD45RA+CD45RO-CCR7+), T-like memory stem cells : TSCM-Like (CD45RA+CD45RO+CCR7+) , effector memory T: TEM (CD45RA-CD45RO+CCR7-), central memory T: TCM (CD45RA-CD45RO+CCR7+), effector T: TE (CD45RA+CD45RO-CCR7-), and others (100%-TSCM-TSCM-Like-TEM-TCM-TE). Exhaustion marker detection method: Flow cytometer detection was performed; antibodies: PD-1: APC, LAG-3 : BV421, TIM-3: Percp5.5.

As shown in FIG. 4 and Tables 15-16, phenotype detection results showed that, compared with the autologous 19CAR, the 44-19CAR and the universal DU-19CAR had a relatively close distribution in each group of typing, the TSCM was slightly high, but TE is slightly lower, however their abilities of tumor killing were not affected according to the killing results. Further, as can be seen from FIG. 5 and Tables 17-18, in the exhaustion marker detection expression data results, compared with the autologous 19CAR, the 44-19CAR and the universal DU-19CAR provided in the present disclosure showed lower expression of PD-1 and LAG-3, also indicating that the 44-19CAR and the universal DU-19CAR provided in the present disclosure had a longer persistence, and was thus conducive to improving the anti-tumor ability. Data corresponding to FIG. 4 was as follows:

**Table 15 Phenotype Data of 44-19CAR**

| | TSCM | | TSCM-like | | TEM | | TCM | | TE | | Others | |
|---|---|---|---|---|---|---|---|---|---|---|---|---|
| CT | 40.81 | 12.65 | 1.62 | 0.47 | 18.86 | 15.94 | 4.94 | 2.5 | 29.23 | 62.62 | 4.54 | 5.82 |
| 19CAR | 51.47 | 14.49 | 8.46 | 0.57 | 10.21 | 18.34 | 16.94 | 2.43 | 7.99 | 58.9 | 4.93 | 5.27 |
| 44-19CAR | 43.52 | 16.51 | 3.77 | 0.55 | 18.44 | 17.99 | 9.37 | 2.38 | 20.7 | 58.18 | 4.2 | 4.39 |

**Table 16 Phenotype Data of DU-44-19CAR**

| | TSCM | | TSCM-like | | TEM | | TCM | | TE | | Others | |
|---|---|---|---|---|---|---|---|---|---|---|---|---|
| CT | 40.81 | 12.65 | 1.62 | 0.47 | 18.86 | 15.94 | 4.94 | 2.5 | 29.23 | 62.62 | 4.54 | 5.82 |
| 19CAR | 51.47 | 14.49 | 8.46 | 0.57 | 10.21 | 18.34 | 16.94 | 2.43 | 7.99 | 58.9 | 4.93 | 5.27 |
| DU-44-19CAR | 47.06 | 19.51 | 1.64 | 1.32 | 12.15 | 14.3 | 8.25 | 2.79 | 24.9 | 55.91 | 6 | 6.17 |

Data corresponding to FIG. 5 was as follows:

**Table 17 Exhaustion Marker of 44-19CAR**

| | CT | | 19CAR | | 44-19CAR | |
|---|---|---|---|---|---|---|
| PD-1 | 17.44 | 16.8 | 9.26 | 10.57 | 16.8 | 10.22 |
| LAG-3 | 45.53 | 53.28 | 56.97 | 58.26 | 46.17 | 51.9 |
| TIM-3 | 0.87 | 0.41 | 0.7 | 0.92 | 1.1 | 0.51 |

**Table 18 Exhaustion Marker of DU-44-19CAR**

| | CT | | 19CAR | | DU-44-19CAR | |
|---|---|---|---|---|---|---|
| PD-1 | 17.44 | 16.8 | 9.26 | 10.57 | 9.48 | 4.85 |
| LAG-3 | 45.53 | 53.28 | 56.97 | 58.26 | 28.31 | 48.64 |
| TIM-3 | 0.87 | 0.41 | 0.7 | 0.92 | 0.96 | 1.84 |

4. Vitality monitoring: the target cells needed to be purified in a sorting manner after electroporation of RNP, the purification was generally performed 5-7 d after activation. Vitality of the cells were monitored from the very same day of sorting, and vitality of the cells at a fixed time point was obtained by counting with a cell counter. In order to maintain experimental consistency, although it was unnecessary to sort out 19CAR and 44-19CAR, monitoring was performed at the time point same as that on DU-44-19CAR.

As shown in FIG. 6 and Tables 19-20, vitality monitoring results indicated that the vitality of the 44-19CAR and the universal DU-19CAR provided in the present disclosure was equivalent to that of 19CAR several days prior to the sorting. However, after 7 days, the vitality of CAR-T cells provided in the present disclosure still remained above 80%, while autologous 19CAR group suffered an obvious decline. Data corresponding to FIG. 6 was as follows:

**Table 19 Vitality of 44-19CAR**

| | CT | 19CAR | 44-19CAR |
|---|---|---|---|
| 0D | 94 | 92 | 96 |
| 2D | 95 | 92 | 96 |
| 4D | 95 | 90 | 92 |
| 7D | 88 | 94 | 94 |
| 10D | 77 | 86 | 92 |

**Table 20 Vitality of DU-44-19CAR**

| | CT | 19CAR | DU-44-19CAR |
|---|---|---|---|
| 0D | 94 | 92 | 98 |
| 2D | 95 | 92 | 92 |
| 4D | 95 | 90 | 95 |
| 7D | 88 | 94 | 95 |
| 10D | 77 | 86 | 90 |

### 5. GVH intensity detection

A microsphere absolute counting method was adopted to measure mixed lymphocyte reaction (MLR), a mixed system of donor cells (CAR-T): recipient cells (PBMC of a healthy human body with different HLA typing from donor cells) at a fixed ratio was configured, and was cultured with 1640 basic culture medium for 8 days, absolute cell counts were detected by a flow cytometry on days 0 and 8, respectively, and relative intensity of GVH was calculated according to changes in the number of cells.

As shown in FIG. 7 and Table 21, GVH intensity detection results indicated that GVH reaction intensity generated by the universal DU-44-19CAR provided in the present disclosure was equivalent to that of the CU-44-19CAR of the conventional universal CAR-T cells, significantly weaker than that of the control group autologous 19CAR, 44-19CAR and CT group. The result indicated that the DU-44-19CAR prepared according to the method in the present disclosure can be used for allogeneic CAR-T immunotherapy, same as that of the conventional universal CAR-T cells. Data corresponding to FIG. 7 was as follows:

**Table 21 Anti-GVH Value of DU-44-19CAR**

| Grouping | GVH reaction degree (%) |
|---|---|
| CT | 80.53 |
| 44-19CAR | 73.18 |
| DU-44-19CAR | 54.9 |
| CU-44-19CAR | 48.93 |

### 7. In vivo efficacy experiment 2 (Comparison between 19CAR and 44-19CAR, between DU-44-19CAR and 19CAR)

Mice were introduced on Day -11, quarantine and other series of detection operations were performed on the mice, and mice were included into a group; target cell tail vein injection of 5e5Nalm6-Luc-GFP was performed on Day 3; random grouping was performed on Day 0, and CAR-T cells were transfused according to a number of effective CAR-T cells of 2e6; and in vivo imaging was then performed to observe tumor clearance in the mice every 7 days.

As shown in FIG. 9, in vivo efficacy results indicated that 19CAR group and 44-19CAR group had significant tumor inhibitory effects in vivo compared with the Control T group; and in vivo anti-tumor effects of the 19CAR group were equivalent to those of the 44 -19CAR group. However, CAR-T survival results indicated that 44-19CAR survived more, further proving that the 44-19CAR provided in the present disclosure could improve the survival of CAR-T cells in vivo, thereby continuously killing tumors.

As shown in FIG. 9, analysis from fluorescence value curve data of the DU-19CAR group and the CU-19CAR group indicated that the anti-tumor effects of the DN-44-19CAR were significantly better than those of the 19CAR group, indicating that the DU-44-19CAR of the universal CAR-T cells provided in the present disclosure was not only universal, but also had better efficacy than the conventional autologous CAR-T cells.

In summary, results of in vitro pharmacodynamic evaluation, GVH intensity detection and in vivo anti-tumor detection on the DU-44-19CAR-T prepared according to the present disclosure indicate that the DU-44-19CAR-T is superior to the conventional universal CAR-T cells in reducing the risk of cytokine release syndrome, improving in vivo survival and anti-tumor effects.

### Example 9. Preparation of T Cells and L-19CAR (Chimeric Antigen Receptor T Cells) Cells Expressing CD62L

CD62L can be combined with a chimeric antigen receptor (CAR) having any target and structure to obtain technical effects disclosed in the present disclosure, a CD19-targeted CAR will be taken as an example for detailed description herein. Similarly, vectors stated herein can be any one of a lentiviral expression vector, a retroviral expression vector, an adenovirus expression vector, an adeno-associated virus expression vector, a DNA vector, an RNA vector, and a plasmid. The lentiviral vector is taken as an example for description herein. Refer to Example 1 for specific preparation steps:

A gene fragment corresponding to the CD62L amino acid sequence was shown as SEQ ID NO 13.

In some embodiments, the T cells co-expressing CD62L and CAR could also be prepared using the method described in Example 6, by replacing CD44 with CD62L only.
Expression of CD62L and CAR was shown in FIG. 24, and both CD62L and CAR can be normally expressed in the T cell.

### Example 10. Preparation of DU-L-19CAR

### 1. Gene knockout experiment

DU-T cells with both CD4 and CD8 knocked out were obtained with reference to Example 1.

2. Method for obtaining DU-L-19CAR: starting sample: peripheral blood of a healthy person. CD4/CD8 single-positive T cells were respectively sorted out through a magnetic bead or a flow cytometry, continuous stimulation was performed for 48 h using Dynameads containing CD3 and CD28 antibodies, a lentivirus containing CAR was infected within 24 h, an RNP complex of sgRNA and Cas9 protein targeting CD4 and CD8 was subjected to electroporation within 48 h, flow cytometry or magnetic bead sorting was performed after electroporation to sort out DU-CAR-T cells, and an culture medium could be 1640 added with an appropriate amount of factors (IL2 and IL4 (and/or) IL7 (and/or) IL15 (and/or) IL21), or any other combination of a culture medium and cytokines suitable for culturing T cells could be used as a culture solution.

Method for obtaining DU-L-19CAR: CD4 and/or CD8 single-positive T cells were obtained, the T cells were infected with a virus containing CD62L and a CAR structure after activation for a plurality of hours, RNP electroporation was performed on the T cells infected with the CAR structure after culture for a plurality of hours, and continuous culture was performed to sort out target cells; or 2. CD4 and/or CD8 single-positive T cells were obtained, electroporation of RNP was performed on the T cells after activation for a plurality of hours, the T cells were infected with a virus containing CD62L and a CAR structure after culture for a plurality of hours, and continuous culture was performed to sort out target cells.

### Example 11. Evaluation of DN-L-19CAR and L-19CAR

### 1. Expression of CAR

Gene editing was performed on CD4 and CD8 of L-19CAR cells, high-purity DU-19CAR cells were sorted out by the magnetic bead, and the operation did not affect expression of CAR structure on T cells. As can be seen from FIG. 24, expression level of the CAR was higher than 70%, and a proportion of the double-negative CAR-T cells after Du-L-19CAR sorting was as high as 100%.

### 2. In vitro killing

A fixed-effect target ratio plate was employed: detailed operation was specified in the kit instructions made by the manufacturer.

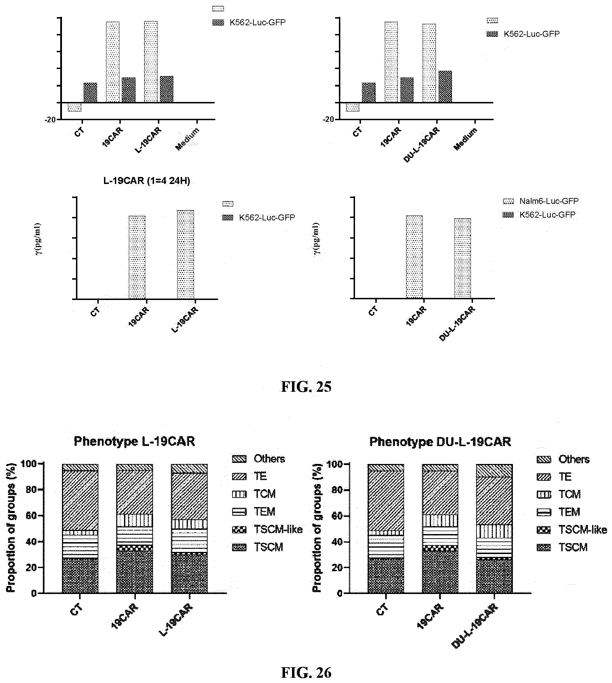

In vitro pharmacodynamic evaluation results were shown in FIG. 25 and Tables 22-25, in vitro efficacy of the L-19CAR and the DU-L-19CAR was equivalent to that of the autologous 19CAR, when an effector-target ratio was 1:4, and killing time was 24 h, killing efficiency thereof could reach 90% or above; and results of cytokine secretion thereof indicated no difference. Data related to FIG. 25 was shown as follows:

**Table 22 Killing Data of L-19CAR**

| | Nalm6-Luc-GFP | K562-Luc-GFP |
|---|---|---|
| CT | -10.63829804 | 22.80829979 |
| 19CAR | 95.12959388 | 29.24838592 |
| L-19CAR | 96.04410065 | 30.96601291 |
| Medium | 0 | 0 |

**Table 23 Killing Data of DU-L-19CAR**

| | Nalm6-Luc-GFP | K562-Luc-GFP |
|---|---|---|
| CT | -10.63829804 | 22.80829979 |
| 19CAR | 95.12959388 | 29.24838592 |
| DU-L-19CAR | 92.77601555 | 37.02034353 |
| Medium | 0 | 0 |

**Table 24 IFN-γ (pg/ml) Secretion Data of L-19CAR**

| | Nalm6-Luc-GFP | K562-Luc-GFP |
|---|---|---|
| CT | 0 | 0 |
| 19CAR | 4080 | 0 |
| L-19CAR | 4342.67 | 0 |

**Table 25 IFN-γ (pg/ml) Secretion Data of DU-L-19CAR**

| | Nalm6-Luc-GFP | K562-Luc-GFP |
|---|---|---|
| CT | 0 | 0 |
| 19CAR | 4080 | 0 |
| DU-L-19CAR | 3939 | 0 |

### 3. Phenotype results

Flow cytometer detection was performed;
as shown in FIG. 26 and Tables 26-27, phenotype detection results showed that, compared with the autologous 19CAR, the L-19CAR and the universal DU-19CAR had a relatively close distribution in each group of typing, the TSCM was slightly low, but the TE was slightly high, which was conducive to the improvement of instantaneous killing of the tumor cells. Data related to FIG. 26 was shown as follows:

**Table 26 Phenotype Data of L-19CAR**

| | TSCM | | TSCM-like | | TEM | | TCM | | TE | | Others | |
|---|---|---|---|---|---|---|---|---|---|---|---|---|
| CT | 40.81 | 12.65 | 1.62 | 0.47 | 18.86 | 15.94 | 4.94 | 2.5 | 29.23 | 62.62 | 4.54 | 5.82 |
| 19CAR | 51.47 | 14.49 | 8.46 | 0.57 | 10.21 | 18.34 | 16.94 | 2.43 | 7.99 | 58.9 | 4.93 | 5.27 |
| L-19CAR | 44.68 | 15.43 | 2.36 | 1.14 | 16.61 | 19.46 | 8.11 | 6.48 | 23.35 | 48.39 | 4.89 | 9.1 |

**Table 27 Phenotype Data of DU-L-19CAR**

| | TSCM | | TSCM-like | | TEM | | TCM | | TE | | Others | |
|---|---|---|---|---|---|---|---|---|---|---|---|---|
| CT | 40.81 | 12.65 | 1.62 | 0.47 | 18.86 | 15.94 | 4.94 | 2.5 | 29.23 | 62.62 | 4.54 | 5.82 |
| 19CAR | 51.47 | 14.49 | 8.46 | 0.57 | 10.21 | 18.34 | 16.94 | 2.43 | 7.99 | 58.9 | 4.93 | 5.27 |
| DU-L-19CAR | 41.37 | 11.13 | 2.62 | 0.96 | 11.91 | 18.4 | 12.29 | 8.69 | 25.07 | 48.52 | 6.74 | 12.3 |

### 4. Exhaustion marker

Exhaustion marker detection method: Flow cytometer detection was performed; antibodies: PD-1: APC, LAG-3 : BV421, TIM-3: Percp5.5.

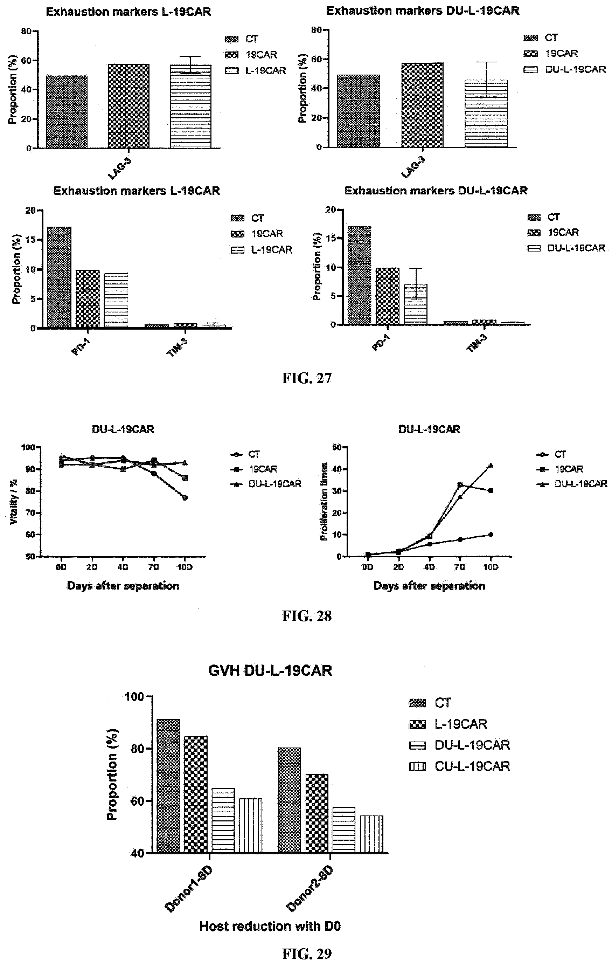

According to expression data results of exhaustion marker detection, as shown in FIG. 27, CARs in the groups thereof did not express TIM-3, but compared with the autologous 19CAR, the L-19CAR and the universal DU-L-19CAR provided in the present disclosure showed lower expression of LAG-3 and PD-1, which also suggested that the L-19CAR and the universal DU-L-19CAR provided in the present disclosure had a longer persistence and a longer time before cell apoptosis, thus being conducive to improving the anti-tumor ability of the CAR-T cells.

5. Proliferation and vitality monitoring: the target cells needed to be purified in a sorting manner after electroporation of RNP, the purification was generally performed 5-7 d after activation. Vitality of the cells were monitored from the very same day of sorting, and vitality of the cells at a fixed time point was obtained by counting with a cell counter. In order to maintain experimental consistency, although it was unnecessary to sort out 19CAR and L-19CAR, monitoring was performed at the same time point same as that on DU-L-19CAR.

Proliferation and vitality monitoring results, as shown in FIG. 28 and Tables 28-29, indicated that proliferation ability and cell viability of the universal DU-L-19CAR provided in the present disclosure were superior to those of the autologous 19 CAR, a proliferation multiple of the DU-L-19CAR provided in the present disclosure was significantly different from that of the other two groups, a proliferation speed thereof was obviously better than that of the autologous 19 CAR group, and cell vitality was also higher than that of the autologous 19 CAR group, indicating that the preparation method in the present disclosure had the effect of remarkably improving CAR-T cell proliferation ability, and could be used for further therapeutic use.

Data related to FIG. 28 was shown as follows:

**Table 28 Vitality of DU-L-19CAR**

| | CT | 19CAR | DU-L-19CAR |
|---|---|---|---|
| 0D | 94 | 92 | 96 |
| 2D | 95 | 92 | 92 |
| 4D | 95 | 90 | 94 |
| 7D | 88 | 94 | 92 |
| 10D | 77 | 86 | 93 |

**Table 29 Proliferation Values of DU-L-19CAR**

| | CT | 19CAR | DU-L-19CAR |
|---|---|---|---|
| 0D | 1 | 1 | 1 |
| 2D | 2.16 | 2.39 | 2.15 |
| 4D | 5.75 | 9.12 | 9.76 |
| 7D | 7.8 | 32.95 | 27.45 |
| 10D | 10.1 | 30.2 | 42 |

### 6. GVH intensity detection

GVH intensity detection results, as shown in FIG. 29 and Table 30, indicated that GVH reaction intensity generated by the double-negative DU-L-19CAR provided in the present disclosure was lower than that of the CU-19CAR of the conventional universal CAR-T cells, and was significantly weaker than that of L-19CAR and 19CAR group, and CT group of the control group autologous CAR-T cells, further indicating that the DU-L-19CAR prepared according to the method in the present disclosure can be used for allogeneic CAR-T immunotherapy, same as that of the conventional universal CAR-T cells.

Data related to FIG. 29 was shown as follows:

**Table 30 Anti-GVH Value of DU-L-19CAR**

| | CT | L-19CAR | DU-L-19CAR | CU-L-19CAR |
|---|---|---|---|---|
| Donor 1-8D | 91.46 | 84.94 | 64.91 | 60.99 |
| Donor2-8D | 80.53 | 70.26 | 57.63 | 54.61 |

### 7. In vivo experiment (Comparison between DU-L-19CAR, L-19CAR and 19CAR)

Mice were introduced on Day 11, quarantine and other series of detection operations were performed on the mice, and mice were included into a group; target cell tail vein injection of 5e5Nalm6-Luc-GFP was performed on Day 3; random grouping was performed on Day 0, and CAR-T cells were transfused according to a number of effective CAR-T cells of 2e6; and in vivo imaging was then performed to observe tumor clearance in the mice every 7 days.

In vivo efficacy results, as shown in FIG. 30, indicated that, compared with the Control T group, the 19CAR groups, DU-L-19CAR groups and L-19CAR had significant tumor inhibitory effects in vivo. Analysis from fluorescence value curve data of the DU-L-19CAR group, the L-19CAR and the 19CAR group indicated that the in vivo efficacy of the DU-L-19CAR group and the L-19CAR were better than that of the 19CAR group, indicating that the L-19CAR group prepared according to the preparation method in the present disclosure had better efficacy. The universal DU-L-19CAR provided in the present disclosure was not only universal, but also had better efficacy than the conventional autologous CAR-T cells, having good inspiration for the field of universal CRA-T cell therapy.

Results of CAR-T copy number in the blood of RT-PCR mice were detected, as shown in FIG. 31, the CAR-T cells of L-19CAR provided in the present disclosure survived in in vivo were more than twice of the conventional autologous 19 CAR group, which was sufficient to illustrate that the method of the present disclosure improved the survival of CAR-T cells in vivo, thereby further improving the continuous anti-tumor ability of CAR-T in vivo.

In summary, results of in vitro pharmacodynamic evaluation, proliferation and vitality monitoring, and GVH intensity detection on the DU-L-19CAR-T prepared according to the present disclosure indicate that the DU-44-19CAR-T is superior to the conventional universal CAR-T cells in terms of anti-tumor effects, cell proliferation ability and cell vitality, and in vivo survival.

The preparation technique for a universal CAR-T cell, and an use of a universal CAR-T cell have been described in detail above. Specific embodiments are used for illustrating principles and implementations of the disclosure herein. The description of the embodiments above is only used for helping understand the method and its core concept of the disclosure. It should be pointed out that those skilled in the art may also make some improvements and modifications without departing from the principle of the present disclosure, and these improvements and modifications should also fall within the scope of protection of the present disclosure.

## Claims

1. An RNP complex, wherein the RNP complex is an RNP complex composed of a Cas9 protein and sgRNA; and the sgRNA is sgRNA of CD4 and/or sgRNA of CD8.

2. The RNP complex according to claim 1, wherein the RNP complex is an intracellular working form of CRISPR/Cas9, and an extracellular delivery mode comprises the following combinations: a plasmid delivery mode, a virus delivery mode, a protein (Cas9) and (RNA) gRNA delivery mode, an mRNA (Cas9) and gRNA delivery mode, an mRNA (Cas9) and plasmid (gRNA) delivery mode, a virus (Cas9) and gRNA delivery mode, a virus (Cas9) and plasmid (gRNA) delivery mode, a plasmid (Cas9) and virus (gRNA) delivery mode, a plasmid (Cas9) and a gRNA delivery mode.

3. The RNP complex according to claim 1 or 2, wherein the CD4 gene is CD4 gene on human chromosome 12: 6,789,528-6,820,799 forward strand; and CD8 gene is CD8A gene on human chromosome 2: 86,784,610-86,808,396 reverse strand, and is CD8B gene on human chromosome 2: 86,815,339-86,861,924 reverse strand.

4. The RNP complex according to claim 1 or 2, wherein a sgRNA sequence of the CD4 is shown as any one of SEQ ID NO. 1-6 or SEQ ID NO. 14-24; and a sgRNA sequence of the CD8 is shown as any one of SEQ ID NO. 7-11 or SEQ ID NO. 25-33.

5. Use of the RNP complex according to any one of claims 1-4 as a promoter of interferon-γ factor secretion level in the allogeneic CAR-T cell.

6. When the therapeutic T cell or CAR-T cell is prepared, an inhibitor or a knockout agent of CD4 gene, and/or an inhibitor or a knockout agent of CD8 gene are used as a cell proliferation agent, a vitality enhancer, a phenotypic distribution improver, and a exhaustion marker expression inhibitor.

7. A T cell using the RNP complex according to claims 1-4 to knock out target sites..

8. The T cell according to claim 7, wherein the CD4 gene and/or CD8 gene of the T cell are/is knocked out and/or inactivated.

9. The T cell according to claim 7 or 8, wherein the T cell expresses CD44 and/or CD62L.

10. The T cell according to any one of claims 7-9, wherein an amino acid sequence of the CD44 is shown as SEQ ID NO. 12, and an amino acid sequence of the CD62L is shown as SEQ ID NO. 13.

11. The T cell according to any one of claims 7-10, wherein the T cell is obtained through gene editing.

12. The T cell according to any one of claims 7-11, wherein the T cell is obtained through natural separation.

13. The T cell according to any one of claims 7-12, the T cell disables or deletes of β2-microglobulin, and/or a common subunit of an HLA-I molecule, and/or CIITA gene.

14. The T cell according to any one of claims 7-13, wherein the T cell is an allogeneic T cell.

15. The T cell according to any one of claims 7-14, wherein the T cell expresses HLA-E and/or HLA-G and/or HLA-F.

16. A preparation method of a T cell according to any one of claims 7-15, wherein a RNP complex according to any one of claims 1-4 is adopted to knock out and/or inactivate a CD4 gene and/or CD8 gene in a T lymphocyte through gene editing technology.

17. The preparation method according to claim 16, wherein any one of the following methods is used to obtain a target cell: (1) a T cell from a fresh or cryopreservation source, CD4 and CD8 genes are knocked out or inactivated through gene editing, the CD4 and CD8 genes can be simultaneously knocked out or inactivated, alternatively, the CD4 or CD8 gene can be first knocked out or inactivated, and the CD8 or CD4 can be further knocked out or inactivated; and the target cell is finally obtained; (2) a CD4-positive T cell is sorted out, and CD4 gene is knocked out or inactivated through gene editing to obtain the target cell; (3) a CD8-positive T cell is sorted out, and CD8 gene is knocked out or inactivated through gene editing to obtain the target cell; (4) a CD4-positive T cell is sorted out, and CD4 gene is knocked out or inactivated through gene editing; a CD8-positive T cell is sorted out, and CD8 gene is knocked out or inactivated through gene editing; and the T cell of which the CD4 gene has been knocked out or inactivated through gene editing and the T cell of which the CD8 gene knocked out or inactivated through gene editing are mixed to obtain the target cell.

18. The preparation method according to claim 16 or 17, wherein the T cell is an activated and/or non-activated T lymphocyte.

19. The preparation method according to any one of claims 16-18, wherein timing for knocking out or inactivating the CD4 gene and/or CD8 gene in the T cell is: after amplification of the T cell, or before amplification of the T cell.

20. The preparation method according to any one of claims 16-19, wherein the gene editing technology is to modify the T cell through electroporation or non-electroporation.

21. The preparation method according to any one of claims 16-20, wherein the gene editing technology comprises one or more of CRISPR, ZFN or TALEN.

22. A T cell obtained through the method according to any one of claims 16-21.

23. A CAR-T cell prepared from the T cell according to any one of claims 7-15 or 22.

24. The CAR-T cell according to claim 23, wherein CAR in the CAR-T cell contains an extracellular domain, a transmembrane domain, and an intracellular signal domain.

25. The CAR-T cell according to claim 23 or 24, wherein the CAR-T cell is an allogeneic CAR-T cell.

26. The CAR-T cell according to any one of claims 23-25, wherein the CAR-T cell expresses membrane-bound IL 15.

27. The CAR-T cell according to any one of claims 23-26, wherein the CAR-T cell further express secretory cytokines, and the cytokines comprise IL2, IL7, IL12, IL21, and IL15.

28. The CAR-T cell according to any one of claims 23-27, wherein the CAR-T cell further expresses and can recognize a fusion protein of molecules in a tumor microenvironment, and an extracellular structure of the fusion protein can recognize one or more of PD1, PDL1, SIRPγ, SIRPδ or TIGIT.

29. The CAR-T cell according to any one of claims 23-28, wherein the CAR structure in the CAR-T cell can be regulated for activation or inactivation, and a structure for regulating activation or inactivation comprise one or more of Peptide neo-epitope (PNE) fluorescein (FITC), 10 amino acids (5B9 tag), FITC-HM-3 bifunctional molecule (FHBM), ScFv, Leucine ZipFv linked to antibody, and Streptavidin 2 (mSA2) biotin-binding domain.

30. The CAR-T cell according to any one of claims 23-29, wherein the CAR-T cell can recognize solid tumors, and hematological tumor cells/tissues; and the antigen recognition region of the CAR-T cell can recognize an antigen comprising one or more of CD 19, CD20, CD22, CD33, CLL-1 (CLEC12A) , CD7, CD5, CD70, CD123, CEACAM5, CEACAM6, CEACAM7, Mesothelin, MUC1, CLDN18.2, CDH17, Trop2, BCMA, NKG2D, PDL1, EGFR, EGFRVIII, PSCA, PSMA, MUC16, CD133, GD2, IL13R2, B7H3, Her2, CD30, SLAMF7, CD38, GPC3, WT1 or TAG-72.

31. A preparation method of the CAR-T cell according to any one of claims 23-30, wherein any one of the following methods can be selected to obtain a target cell:
(1) collecting and obtaining the T cell, infecting the T cell with a virus containing CAR structure, performing RNP electroporation of the T cell infected with a CAR structure to obtain the target cell; or
(2) collecting and obtaining the T cell, performing electroporation of the T cell and then infecting the T cell with a virus containing a CAR structure to obtain the target cell.

32. The preparation method according to claim 31, wherein any one of the following methods can be selected to obtain a target cell:
( 1) collecting and obtaining the T cell, infecting the T cell with viruses containing CD62 and a CAR structure, or infecting the T cell with viruses containing CD44 and a CAR structure; and performing RNP electroporation of the T cell infected with the CAR structure to obtain the target cell; or
( 2) collecting and obtaining the T cell, performing electroporation RNP of the T cell and then infecting the T cell with viruses containing CD44 and a CAR structure, or infecting the T cell with viruses containing CD62 and a CAR structure to obtain the target cell.

33. The preparation method according to claim 32, wherein any one of the following methods can be selected to obtain a target cell:
(1) collecting and obtaining the T cell, infecting the T cell with viruses containing CD62L, CD44 and a CAR structure, performing RNP electroporation of the T cell infected with the CAR structure to obtain the target cell; or
(2) collecting and obtaining the T cell, performing electroporation RNP of the T cell and then infecting the T cell with viruses CD62L, CD44 and a CAR structure to obtain the target cell.

34. The preparation method according to any one of claims 31-33, wherein the RNP complex in the process of RNP electroporation is the RNP complex according to claim 1.

35. The preparation method according to any one of claims 31 - -34, wherein a molar ratio of the Cas9 protein to the sgRNA is 1: 2.

36. A recombinant vector for infecting the CAR-T cell according to any one of claims 23-30 or the CAR-T cell prepared by the preparation method according to any one of claims 31-35, wherein the recombinant vector contains a CAR structure.

37. The recombinant vector according to claim 36, wherein the recombinant vector contains a CD44 gene functional fragment and/or a CD62L gene functional fragment.

38. The recombinant vector according to claim 36 or 37, wherein the recombinant vector contains:
(1) CD44 and a CAR structure; or
(2) CD62L and a CAR structure; or
(3) CD62L, CD44 and a CAR structure.

39. The recombinant vector according to any one of claims 36-38, wherein the expression vector selected from the recombinant vector is one or more of a lentiviral expression vector, a retroviral expression vector, an adenovirus expression vector, an adeno-associated virus expression vector, a DNA vector, an RNA vector, and a plasmid.

40. A cell composition containing the T cell according to any one of claims 7-15 or claim 22 and/or the CAR-T cell according to any one of claims 23-30 or the CAR-T cell prepared by the preparation method according to any one of claims 31-35.

41. The cell composition according to claim 40, wherein a proportion of the T cell or the CAR-T cell contained in the cell composition is not less than 90%, 80%, 70% or 60% by quantity.

42. The cell composition according to claim 40 or 41, wherein the cell composition may be a CIK cell or a NKT cell induced by activation.

43. An use the T cell according to any one of claims 7-15 or claim 22 and/or the CAR-T cell according to any one of claims 23-30 or the CAR-T cell prepared by the preparation method according to any one of claims 31-35 and/or the recombinant vector according to any one of claims 36-39 or the cell composition according to any one of claims 40-42 in preparing cell therapy drugs.

44. The use according to claim 43, wherein the use of the T cell according to any one of claims 7-15 or claim 22 and/or the CAR-T cell according to any one of claims 23-30 or the CAR-T cell prepared by the preparation method according to any one of claims 31-35 and/or the recombinant vector according to any one of claims 36-39 or the cell composition according to any one of claims 40-42 in preparing drugs for the treatment of acute lymphoblastic leukemia, chronic lymphocytic leukemia, chronic myelogenous leukemia, non-Hodgkin lymphoma, Hodgkin lymphoma, prostate cancer, colorectal cancer, breast cancer, ovarian cancer, cervical cancer, pancreatic cancer, lung cancer, kidney cancer, liver cancer, brain cancer and/or skin cancer.

45. An use of CD62L and/or CD44 in preparing a vitality enhancer for the CAR-T cell.

46. An use of CD44 combined with CD62L in preparing a vitality enhancer for the CAR-T cell.

47. CD62L and/or CD44 are used to prepare an inhibitor of PD-1 and LAG-3 factors secreted by the CAR-T cell.

48. When preparing the CAR-T cell, CD62L and/or CD44 genes are used as accelerators for tumor cell killing, and the CAR-T cell is the conventional CAR-T cell, the CAR-T cell or the universal CAR-T cell without expressing CD4 and/or CD8 genes.
